# FASCICULE DE BREVET EUROPEEN

(11) **EP 1 625 397 B1**
(45) Date de publication et mention de la délivrance du brevet: **11.04.2007**
(21) Numéro de dépôt: 04742867.7
(22) Date de dépôt: 14.05.2004
(51) Int. Cl.: G01N 33/53

(54) **PROCEDE DE DETECTION DE FLUORURE OU DE FLUORURE D'HYDROGENE ET TROUSSE DE DETECTION**
NACHWEISVERFAHREN VON FLUORID ODER HYDROGENFLUORID UND NACHWEISKIT
METHOD FOR THE DETECTION OF FLUORIDE OR HYDROGEN FLUORIDE AND DETECTION KIT

(30) Priorité: 20.05.2003 FR 0350160; 22.05.2003 FR 0350167
(43) Date de publication de la demande: 15.02.2006
(73) Titulaire: COMMISSARIAT A L'ENERGIE ATOMIQUE, 75015 Paris (FR)
(72) Inventeur: EZAN, Eric, F-92240 MALAKOFF (FR); SAGOT, Marie-Astrid, F-91150 ABBEVILLE LA RIVIERE (FR); PRADELLES, Philippe, F-91140 VILLEBON SUR YVETTE (FR)
(74) Mandataire: Poulin, Gérard
(86) Numéro de dépôt international: PCT/FR2004/050194
(87) Numéro de publication internationale: WO 2004/104579

(56) Documents cités:
- WO-A-03/065408
- DESCALZO, ANA B ET AL: "A new method for fluoride determination by using fluorophores and dyes anchored onto MCM-41." CHEM. COMMU., vol. 6, 11 mars 2002 (2002-03-11), pages 562-563, XP002275945
- PATENT ABSTRACTS OF JAPAN vol. 2003, no. 06, 3 juin 2003 (2003-06-03) & JP 2003 043025 A (TOHOKU TECHNO ARCH CO LTD), 13 février 2003 (2003-02-13)

## Description

### DOMAINE TECHNIQUE

La présente invention se rapporte à un procédé de détection et/ou de dosage de fluorure (F⁻) ou de fluorure d'hydrogène (HF) présent dans un échantillon, ainsi qu'à une trousse de détection pour la mise en oeuvre de ce procédé. Cette invention permet une mesure efficace et sensible d'un polluant environnemental. Elle est basée sur un procédé tout à fait original et simple à mettre en oeuvre.

Le fluorure d'hydrogène est un acide inorganique fort, incolore et très soluble dans l'eau où il forme l'acide fluorhydrique. HF est un gaz largement utilisé en milieu industriel, notamment pour la production de polymères, de liquides de refroidissement, de produits pour éteindre des incendies, pour l'affinage de l'aluminium, la préparation de combustibles nucléaires et la fabrication d'éléments pour l'électronique. En outre, il s'agit d'un produit émis lors de la combustion de charbon, de déchets ménagers ou industriels, et de plastiques.

HF est toxique à partir de concentrations de l'ordre de 3x10⁻² l HF / 10⁶ l d'air (30 ppm). Il s'agit d'un irritant puissant, qui peut provoquer des brûlures par exposition cutanéo-muqueuse ainsi qu'une inflammation des voies aériennes supérieures et inférieures. Son absorption peut en outre provoquer des troubles métaboliques.

Par ailleurs, ce produit est très corrosif vis-à-vis de nombreux matériaux tels que le fer, le bronze et le verre.

La protection de l'homme, de l'environnement, ainsi que des matériels industriels nécessite en conséquence la mise en place de moyens de contrôle de sa concentration notamment dans les effluents industriels, dans les laboratoires, ainsi que dans l'atmosphère entourant ces installations, afin de pouvoir prendre des mesures appropriées en cas de détection de concentrations dangereuses en HF.
Des normes de concentration atmosphériques de HF acceptables ont été établies dans de nombreux pays. Elles se situent généralement entre 5x10⁻⁵ et 5×10⁻⁴ l de HF / 10⁶ l d'air (entre 0,05 et 0,5 ppm).

Dans la suite de la description, les références entre crochets [ ] renvoient à la liste de référence annexée à la présente.

### Art antérieur

Pour la mesure du HF ou des ions fluorures, de nombreuses méthodes ont été développées et décrites à ce jour. Ces techniques sont généralement basées sur une détection chimique, électrochimique, spectrométrique ou optique. Il existe de ,nombreux dispositifs disponibles dans le commerce qui permettent de détecter et de mesurer la quantité de HF.

Le document [1] de la liste de référence annexée décrit un certain nombre de méthodes électrochimiques, spectrophotométriques, chromatographiques utilisables pour détecter et mesurer la quantité de HF dans un échantillon.

Une méthode de référence, appelée méthode ID-110, basée sur l'utilisation d'électrodes spécifiques est décrite sur le site Internet de l'OSHA référencé [2]. La sensibilité de cette méthode est de 1, 2x10⁻² l de HF / 10⁶ l d'air (12 ppb), mais il s'agit d'un test de laboratoire difficilement applicable au terrain. Les méthodes de terrain sont quant à elles beaucoup moins sensibles et de l'ordre de 0,2 l de HF / 10⁶ l d'air (200 ppb).

Une autre méthode utilise un détecteur basé sur un substrat de carbure de silicium (« Metal Insulator Semiconductor »: MIS). Elle est décrite par exemple dans le document référencé [3]. Divers fabricants proposent divers appareils électroniques de détection pour mesurer le HF. Il s'agit par exemple de l'appareil OEM Fluorine Sensor (marque de commerce) de la société Bionics Instrument, décrit sur le site Internet référencé [4]; ou de l'appareil LaserGas (marque de commerce) de la société NORSK ELEKTRO OPTIKK A/S.
Les limites de détection annoncées pour ces appareils sont exprimées en partie par million ou par billion (milliard en français soit 10⁹) (ppm ou ppb) pour un milieu gazeux, ou nano ou microgrammes par millilitre (ng/ml ou µg/ml) pour un milieu aqueux. Elles sont en général de l'ordre de 10⁻⁴ l de HF / 10⁶ l d'air (0,1 ppm) ou en solution de 1 à 1000 ng de HF/ml pour les meilleurs systèmes.

Malheureusement ces méthodes et appareils nécessitent de la place, sont difficilement mobiles, sont parfois difficiles à mettre en oeuvre, et souvent coûteux.

Un « moyen de détection idéal » du HF devrait combiner les caractéristiques suivantes :
- la sensibilité : mesure d'au moins de 10⁻⁴ l de HF / 10⁶ l d'air (0,1 ppm),
- le coût : il ne doit pas être trop élevé afin de pouvoir, si nécessaire, multiplier facilement le nombre de détecteurs,
- la disponibilité : un certain nombre de techniques publiées ne sont pas disponibles sur le marché, notamment du fait de leur difficulté de mise en oeuvre,
- la mobilité: il devrait pouvoir être déplacé facilement pour pouvoir être utilisé à différents endroits,
- la rapidité de mise en oeuvre et d'exploitation des résultats.

Il semble qu'aucune des méthodes ou dispositifs de l'art antérieur combine l'ensemble de ces propriétés.

Il existe donc un réel besoin de nouvelles techniques qui combinent le plus possible les caractéristiques précitées du « moyen de détection idéal ».

### Exposé de l'invention

L'objectif atteint par la présente invention est précisément de fournir un procédé et une trousse de détection de HF qui présentent toutes les caractéristiques précitées, et qui en outre ne présentent pas les inconvénients précités des procédés et dispositifs de l'art antérieur.

Cet objectif est atteint au moyen d'un procédé de détection et/ou de dosage de fluorure (F⁻) ou de fluorure d'hydrogène (HF) dans un échantillon comprenant les étapes suivantes:
- mettre en contact en solution aqueuse ledit échantillon avec un composé organique silylé pour obtenir une solution de mesure, ledit composé organique silylé étant désilylé lorsqu'il est en présence d'acide fluorhydrique ou de fluorure, le composé organique silylé et le composé organique désilylé pouvant être détectés et/ou dosés distinctement l'un de l'autre; et
- détecter et/ou doser dans ladite solution de mesure l'apparition du composé organique désilylé ou la disparition du composé organique silylé qui a lieu si du fluorure ou du fluorure d'hydrogène est présent dans l'échantillon.

Dans la description qui suit, le fluorure (F⁻), le fluorure d'hydrogène (HF) et l'acide fluorhydrique sont implicitement désignés par les termes « fluor » ou « fluor et ses dérivés ». Par fluorure(s), on entend les sels du fluor.

Le fluor est un atome très nucléophile, et de ce fait, il peut intervenir dans des réactions de substitution nucléophile. Plus particulièrement, il peut attaquer de manière spécifique des liaisons de type silicium-oxygène (Si-O) de la manière indiquée dans l'équation chimique ci-dessous.

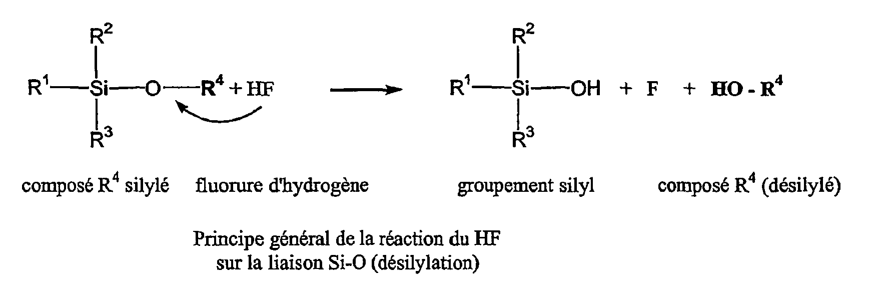

Dans cette équation chimique R¹, R² et R³ sont des substituants de Si, et forment avec ce dernier un groupement de silylation du composé organique R⁴ au sens de la présente invention.

R¹, R² et R³ peuvent être choisis indépendamment parmi des alkyles en C₁ à C₆. Par exemple R¹, R², R³ peuvent être choisis indépendamment dans le groupe constitué du méthyle, de l'éthyle, du propyle ou du butyle.

Dans cette équation chimique, -R⁴ représente donc le composé organique silylé (à gauche) ou désilylé (à droite) dans la mise en oeuvre du procédé de l'invention. Ce composé, pour pouvoir être silylé comprend au moins une fonction hydroxyle accessible pour sa silylation. Ce composé est avantageusement un composé organique afin de faciliter sa détection lors de la mise en oeuvre du procédé de l'invention. Il a en général un poids moléculaire de 250 à 200000 g.mol⁻¹, par exemple de 250 à 1500 g.mol⁻¹, notamment pour des questions de solubilité et donc de sensibilité et de reproductibilité des résultats de détection et/ou de dosage du fluor. Il peut s'agir d'un composé hydroxylé choisi par exemple parmi l'estradiol, des peptides, par exemple des peptides comprenant de 3 à 50 résidus d'acide aminés, l'acide homovanillique, l'amphotéricine, les stéroïdes, les cytokines, l'acide arachidonique, ou des dérivés de ceux-ci.

Un peptide utilisable dans la présente invention est décrit par exemple dans la référence [5] ; de l'acide homovanillique utilisable et des dérivés de celui-ci sont décrits par exemple dans les références [6] et [7] ; de l'amphotrécine utilisable est décrite par exemple dans les références [8] à [10] ; des stéroïdes et dérivés de stéroïdes utilisables dans la présente invention sont décrits par exemple dans les références [11] à [17] ; des cytokines et des dérivés de cytokines utilisables dans la présente invention sont décrites par exemple dans les références [18] à [20] ;.et de l'acide arachidonique et ses dérivés utilisables sont décrits par exemple dans les références [21] à [24].

L'essentiel est que le composé organique silylé et le composé organique désilylé puissent être détectés et/ou dosés distinctement l'un de l'autre afin de pouvoir détecter et/ou doser la disparition de l'un et/ou l'apparition de l'autre de ces composés dans la solution de mesure. Le procédé de l'invention repose en effet sur la détection du composé organique silylé ou du composé organique désilylé, ou de ces deux composés, mais distinctement.

Selon l'invention, tout réactif approprié peut être utilisé pour fixer un groupe silyl (silylation) sur le composé organique choisi, pourvu que le composé organique désilylé, ou forme native du composé organique, puisse être retrouvé intact par simple action du fluor ou de ses dérivés sur le composé organique silylé. Les groupes ou fonctions silyl utilisables sont décrits ci-dessus, dans l'équation chimique.

On peut citer à titre d'exemple les réactifs suivants utilisables pour silyler des composés organiques hydroxylés pour la mise en oeuvre de la présente invention : le N,Obis(triméthylsilyl)trifluoroacétamide (ou BTSFA), le N-méthyl-N-(tert-butyl-diméthylsilyl)-trifluoroacetamide (ou MTBSTFA), le triméthylsilyl (ou TMS), le t-butyldiméthylsilyl (ou t-BDMS), N, le OBis(triméthylsilyl)acetamide (ou BSA), l'hexaméthyldisiazane (ou HMDS), le N-Méthyltriméthylsilyltrifluoroacetamide (ou MSTFA), le Trimethylchlorosilane (ou TMCS), le trimethylsilylimidazole (ou TMSI), etc.

D'autres réactifs et modes opératoires de silylation utilisables pour la mise en oeuvre de la présente invention pourront encore être trouvés par exemple dans les références [25] à [30] de la liste de référence annexée.

Ces réactifs sont en général d'utilisation simple et courante et permettent de transférer une fonction silyl (contenant un atome de silicium) sur une fonction hydroxyle du composé organique choisi.

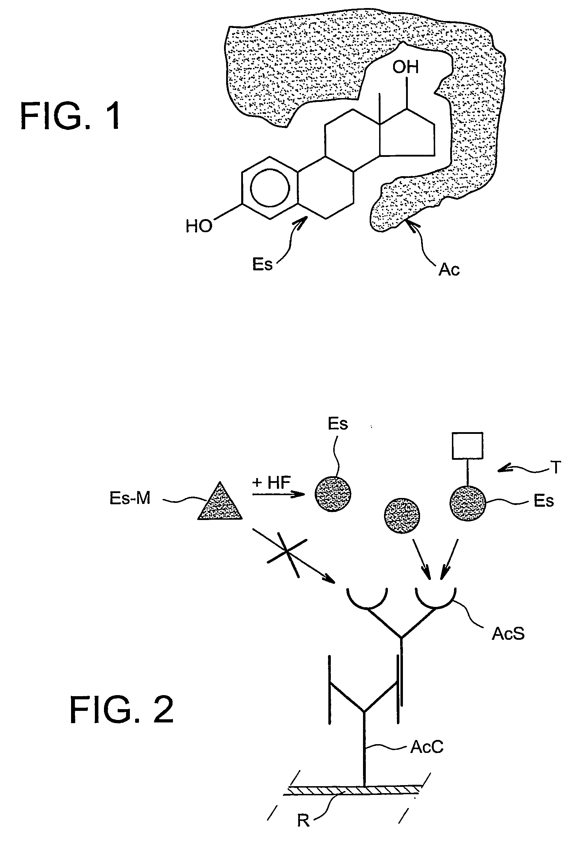

On peut citer en particulier les deux réactifs suivants pour illustrer le fait que les inventeurs ont en outre mis en évidence, dans la présente invention, une variation de sensibilité et de sélectivité de détection et/ou dosage du fluor selon l'invention en fonction du groupement silyl utilisé:
- Le BTSFA qui greffe une fonction triméthylsilyl (-Si(CH₃)₃) sur les groupements -OH du composé organique choisi.
- Le MTBSTFA qui greffe une fonction diméthyltertbutylsilyl (Si(CH₃)₂C(CH₃)₃) sur les groupements -OH du composé organique choisi.
   Le BTSFA présente l'avantage de transférer sur les composés hydroxylés un silyl peu hydrophobe, permettant une assez bonne solubilité du composé organique silylé. Cependant les composés silylés obtenus ne présentent pas toujours de spécificité vis à vis de HF pour leur transformation en composés désilylés dans la mise en oeuvre du procédé de l'invention. Ils peuvent également être transformés en composés désilylés par d'autres acides présents dans l'échantillon. Ce réactif peut donc être utilisé de préférence pour détecter du fluor où la spécificité par rapport au fluor n'est pas requise, ou alors pour détecter du fluor dans des échantillons dans lesquels la concentration des autres acides est assez faible pour ne pas perturber la détection ou le dosage du fluor.
   Le MTBSTFA quant à lui transfert un groupement silyl plus hydrophobe sur les composés hydroxylés et la solubilité des composés silylés obtenus en milieu aqueux est moins bonne que pour les composés silylés obtenus avec le BTSFA. En revanche, de manière tout à fait inattendue, la désilylation des composés silylés par MTBSTFA est plus spécifique du HF que des autres acides testés comme cela est montré dans les exemples. Ce réactif peut donc être avantageusement utilisé pour détecter du fluorure d'hydrogène dans un échantillon lorsque la spécificité par rapport au fluor est requise, par exemple lorsque l'échantillon comprend d'autres acides à des concentrations non négligeables.
   Les inventeurs ont ainsi découvert, notamment à travers l'utilisation de ces réactifs, que la spécificité de l'attaque de la liaison Si-O par le fluor dépend aussi des radicaux fixés sur les atomes de silicium et d'oxygène. Ainsi, en choisissant de manière appropriée le groupement de silylation, les autres acides inorganiques ou organiques et sels de l'échantillon n'interfèrent pas sur les mesures effectuées dans la mise en oeuvre du procédé de l'invention. Par exemple, HF a à peu près la même activité de désilylation que HCl lorsque le procédé de l'invention est mis en oeuvre avec -Si-(CH₃)₃ et une activité supérieure d'environ 1000 fois (pour HF par rapport à HCl) lorsque le procédé de l'invention est mis en oeuvre avec -O-Si-(CH₃)₂-C(CH₃)₃.
   Comme l'indique l'équation chimique ci-dessus, la transformation du composé organique silylé en composé organique désilylé par le fluor ou ses dérivés est proportionnelle à la quantité de HF présente dans l'échantillon. Il est donc possible, grâce au procédé de l'invention à la fois de détecter et de doser le fluor et ses dérivés présent dans un échantillon. La caractéristique essentielle de l'invention et le caractère inventif consistent précisément à effectuer une détection du fluorure et/ou du fluorure d'hydrogène en utilisant un moyen de détection qui fait la différence entre le composé organique silylé et le composé organique non silylé (désilylé), et donc de détecter/quantifier la désilylation catalysée par la présence de fluor.
   L'étape de mise en contact peut être réalisée par mélange de l'échantillon et du composé organique silylé dans une solution aqueuse. Le mélange peut être réalisé par simple addition de l'échantillon et du fluor en solution, ou par un mélange actif par exemple au moyen d'un agitateur mécanique, magnétique, à ultrasons, etc. de l'échantillon et du fluor en solution. L'objectif étant bien entendu de favoriser l'interaction entre le fluor s'il est présent et le composé organique silylé pour qu'il réagissent ensemble.
   L'échantillon peut être un échantillon liquide, solide ou gazeux. Dans les cas où l'échantillon n'est pas liquide, l'étape de mise en contact en solution aqueuse de échantillon avec le composé organique silylé du procédé de l'invention comprend évidemment une mise en solution aqueuse de l'échantillon, par exemple par barbotage lorsque l'échantillon est gazeux ou par mélange ou solubilisation sous agitation lorsque l'échantillon est solide, le but de cette mise en solution étant de retrouver le fluor de l'échantillon gazeux ou solide dans la solution aqueuse de mise en contact. Les procédés utilisés pour cette mise en solution sont bien connus de l'homme du métier.
   Les inventeurs ont mis en évidence que l'addition d'un solvant organique miscible à l'eau à ladite solution aqueuse de mise en contact peut augmenter de façon importante la sensibilité de détection et/ou de dosage du fluor par le procédé de l'invention. A titre d'exemple, le solvant organique peut être choisi parmi le diméthylformamide (DMF), le diméthylsulfoxide (DMSO), l'éthanol, le méthanol ou des solvants organiques équivalents connus de l'homme du métier. En effet, les inventeurs ont mesuré une augmentation de la sensibilité de détection du fluor d'un facteur de 100 et même de 500 en utilisant de tels solvants. Par exemple, avec le DMSO, la sensibilité de détection va jusqu'à 0,001µg/ml.
   Le solvant organique pourrait avoir plusieurs effets différents: faciliter la solubilité du composé organique silylé, faciliter la désilylation, diminuer les interférences dans la détection (noté notamment dans les immunotests). Quoi qu'il en soit, un seul de ces effets ne suffit pas à expliquer l'augmentation significative tout à fait surprenante de la sensibilité de détection en présence du solvant organique. Ce solvant organique peut être présent en une quantité allant de 1 à 99% en volume de la solution aqueuse de mise en contact, avantageusement de 50 à 95% en volume de la solution aqueuse de mise en contact, le reste étant de l'eau.
   Ce solvant peut avoir avantageusement pour origine le solvant de récupération du composé organique silylé après sa fabrication, c'est à dire après la silylation du composé organique. L'étape de mise en contact peut alors être réalisée par mélange de la solution aqueuse de fluor et de la solution organique du composé organique silylé. Autrement, le solvant organique peut être ajouté indépendamment dans la solution de mise en contact.
   La détection du composé organique silylé qui disparaît ou du composé organique désilylé qui apparaît peut être réalisée par tout moyen connu de l'homme du métier permettant de mettre en évidence distinctement l'un ou l'autre de ces composés. Il peut s'agir par exemple d'une détection et/ou d'un dosage réalisé par chromatographie en phase gazeuse ou d'une détection et/ou d'un dosage réalisé au moyen d'un test immunologique.
   De manière générale, pour le dosage, une gamme d'étalonnage peut être au préalable réalisée en appliquant le procédé de l'invention avec des quantités connues d'acide fluorhydrique ou de fluor, ou avec des quantités connues du composé organique silylé ou non silylé. Cette gamme d'étalonnage permettra alors, par simple extrapolation, de déterminer la quantité de fluor, de fluorure d'hydrogène ou d'acide fluorhydrique présente dans l'échantillon.
   Dans un premier mode de réalisation de la présente invention, la détection et/ou le dosage peut être réalisé par chromatographie en phase gazeuse. En effet, cette technique permet de détecter et/ou doser distinctement la forme silylé ou la forme désilylé du composé organique selon l'invention, et donc le fluor s'il est présent. Les techniques de chromatographie utilisables sont celles connues de l'homme du métier. A titre d'exemple, on peut citer les techniques décrites dans les références [31] à [33] de la liste de références annexée.
   Les composés organiques utilisables dans ce premier mode de réalisation peuvent être les composés organiques hydroxylés précités. Les techniques et réactifs de silylation peuvent également être ceux précités.
   Dans un deuxième mode de réalisation de la présente invention, la détection et/ou le dosage de l'apparition du composé organique non silylé ou de la disparition du composé organique silylé est avantageusement réalisée au moyen d'un test immunologique, c'est à dire en utilisant des anticorps dirigés soit contre composé organique non silylé soit contre le composé organique silylé. Aucune approche de ce genre n'apparaît dans l'art antérieur. En outre, ce mode de réalisation permet une détection beaucoup plus sensible du fluor que les techniques de l'art antérieur.
   D'une manière générale, les anticorps sont des protéines capables de reconnaître et de se lier avec une très grande spécificité à des structures appelées antigènes pour former un complexe anticorps-antigène détectable. Cependant, HF étant une molécule de très petite taille, il est impossible de produire des anticorps contre celui-ci. Ne pouvant produire des anticorps dirigés directement contre le HF, les inventeurs ont adopté une stratégie originale basée sur les propriétés chimiques précitées du fluor et sur l'utilisation de composés organiques silylé particuliers présentant la particularité d'entraîner la génération d'anticorps lorsqu'ils sont injectés à un animal.
   Les composés organiques utilisables dans ce deuxième mode de réalisation sont des composés organiques présentant un ou plusieurs groupement(s) hydroxyles qui peuvent permettre la fixation d'un groupement silyl au sens de la présente invention, et qui induisent chez un animal dans lequel ils sont injectés la fabrication spécifique d'anticorps dirigés contre le composé organique silylé ou contre le composé organique non silylé. Ils ont en général un poids moléculaire de 250 à 200000 g.mol⁻¹, par exemple de 250 à 1500 g.mol⁻¹. Il peut s'agir par exemple d'un composé organique choisi parmi les composés organiques hydroxylés cités ci-dessus, par exemple choisi parmi l'estradiol ; des peptides, par exemple des peptides comprenant de 3 à 50 résidus d'acide aminés, par exemple le tétrapeptide Ser-Asp-Lys-Pro acétylé (AcSDKP) cité dans la référence [5] ; l'acide homovanillique ; l'amphotéricine ; les stéroïdes ; les cytokines ; l'acide arachidonique ; ou des dérivés de ces composés. Il peut s'agir par exemple de composés tels que ceux cités dans les références [5] à [24].
   A tire d'exemple illustratif, on peut utiliser de l'estradiol ou ses dérivés. Selon l'invention, par « estradiol » on entend par exemple l'estratriène-1, 3, 5 diol-3, 17µ ou 17µ, ou leurs dérivés, ou d'autres composés équivalents pourvu qu'ils soient capables de générer la formation d'anticorps lorsqu'ils sont injectés à un animal, par exemple une souris, et qu'ils puissent être silylés, au sens de la présente invention.
   Des exemples d'estradiols silylés obtenus par les inventeurs et utilisables dans la présente invention sont représentés ci-dessous. L'estradiol présente des fonctions -OH sur lesquelles il est possible de greffer un groupement silyl, au moyen de liaisons Si-O, comme représenté ci-dessous (à comparer par exemple avec l'estradiol natif (Es) représenté sur la figure 1 annexée).
   Les réactifs utilisables pour la silylation du composé organique choisi pour la mise en oeuvre de ce deuxième mode de réalisation de la présente invention, peuvent être ceux indiqués ci-dessus, par exemple le BTSFA ou le MTBSTFA.
   Les anticorps utilisables dans ce mode de réalisation de l'invention présentent la particularité de reconnaître différemment le composé organique silylé et le composé organique non silylé. Ces anticorps peuvent être monoclonaux. Ils peuvent être préparés par les techniques habituelles de préparation d'anticorps de ce type, par exemple par injection du composé organique silylé ou bien du composé organique non silylé chez la souris pour obtenir un anticorps de souris spécifique d'un de ces composés seulement. Des tests peuvent être réalisés sur différents lots d'anticorps fabriqués pour sélectionner des anticorps spécifiques d'un seul des deux composés (silylé ou non silylé) pour la mise en oeuvre du procédé de l'invention. L'homme du métier connaît ces techniques de fabrication d'anticorps.

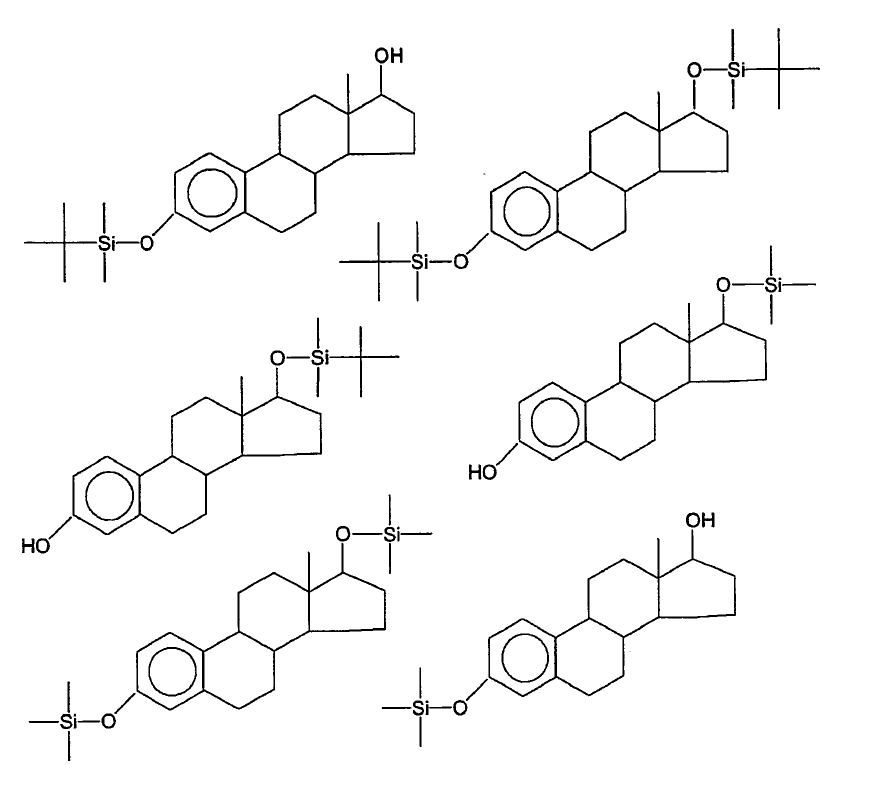

### Exemples d'estradiols silylés utilisables dans la présente invention

A titre d'exemple, les documents référencés [34] à [38] exposent des modes opératoires appropriés de fabrication d'anticorps utilisables pour la mise en oeuvre de la présente invention.

A titre d'exemples également, les références [5] à [24] et [39] à [42] décrivent en outre des anticorps utilisables pour la mise en oeuvre de la présente invention lorsque le composé organique est un composé silylable choisi dans la liste ci-dessus.

Dans l'exemple illustré par la figure 1 annexée, l'anticorps dirigé contre l'estradiol utilisé dans l'immunodosage est choisi de sorte qu'il ne reconnaisse plus, ou beaucoup moins bien (de manière suffisante pour que cette différence de reconnaissance puisse être détectée), celui-ci lorsqu'il est modifié par un groupement silyl (appelé aussi, ci-après, « estradiol modifié »). L'action du fluor sur l'estradiol modifié attaque la liaison Si-O (réaction de désilylation) et permet la réapparition de l'estradiol natif, qui peut alors être reconnu par son anticorps spécifique, appelé ci-dessous « premier anticorps ».

Tous les moyens de détection d'une reconnaissance antigène/anticorps connus de l'homme du métier peuvent être utilisés dans la présente invention pour détecter soit la reconnaissance du composé organique désilylé (« composé organique natif ») par son anticorps, soit la reconnaissance du composé organique silylé par son anticorps. C'est pourquoi les termes « moyen permettant de détecter une interaction entre un premier anticorps et le composé organique non silylé » ou « moyen permettant de détecter une interaction entre un premier anticorps et le composé organique silylé » sont utilisés dans la présente. Les conditions opératoires de telles détections utilisant des anticorps sont connues de l'homme du métier. Des protocoles, réactifs, tampons et conditions opératoires utilisables sont décrits par exemple dans les documents référencés [5] à [24] et [39] à [42].

La détection suivant ce mode de réalisation peut être réalisée suivant un immunodosage de type « compétitif » ou de type « non compétitif », par exemple tels que ceux habituellement utilisés dans les procédés de détection et/ou dans les procédés de dosage immunologique.

Selon une première variante de ce deuxième mode de réalisation de la présente invention, le moyen de détection peut être par exemple une molécule marquée, appelée « traceur », entrant en compétition avec le composé organique silylé ou non silylé, par exemple l'estradiol silylé ou non silylé, vis à vis dudit premier anticorps. Cette molécule marquée peut utiliser une enzyme, par exemple l'acétylcholine estérase, un marqueur fluorescent, un marqueur luminescent ou un radio-isotope pour la détection.

L'immunodosage (ou dosage immunologique) du composé organique silylé ou non silylé, est alors dit « par compétition » . : il fait intervenir un anticorps dirigé contre le composé organique silylé ou non silylé, par exemple l'estradiol silylé ou non silylé, présent dans une gamme d'étalonnage ou dans un échantillon, et un traceur chimiquement proche du composé organique selon l'invention, par exemple de l'estradiol, et porteur d'un signal (marqueur), par exemple un signal enzymatique, fluorescent, luminescent ou radioactif. Dans l'exemple précité de l'estradiol, le traceur peut être obtenu par exemple en couplant l'estradiol avec une enzyme, par exemple l'acétylcholine estérase.

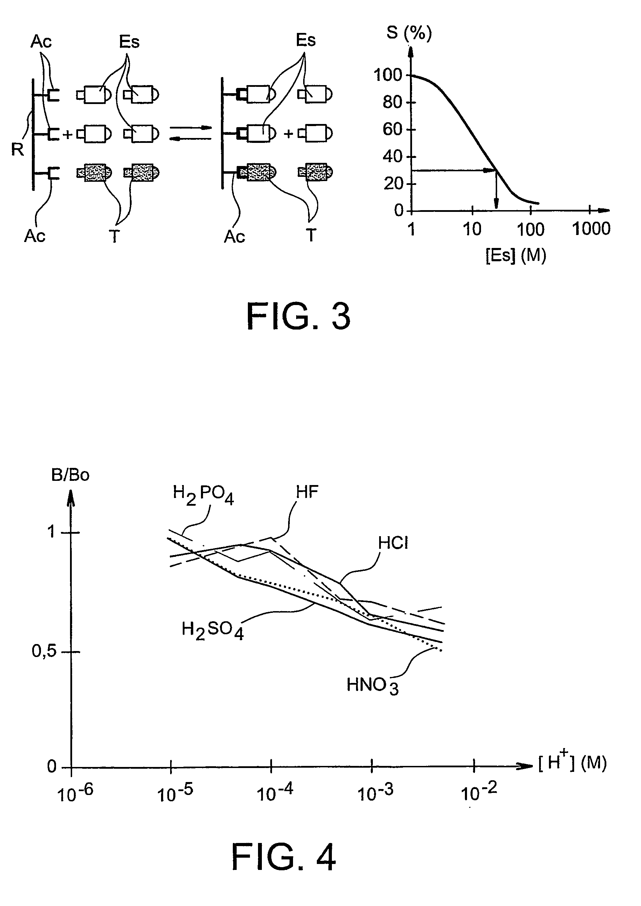

Une détection « par compétition » utilisable pour mettre en oeuvre le procédé de l'invention est représenté schématiquement sur les figures 2 et 3. Le composé organique utilisé sur ces figures est l'estradiol. La molécule marquée ou traceur est référencée « T », l'estradiol non silylé (non modifié) est référencé « Es » et l'estradiol silylé (modifié) « Es-M ». Le premier anticorps est par exemple un anticorps de souris (AcS) La figure 2 montre que l'estradiol modifié (Es-M) est incapable d'être reconnu par l'anticorps (AcS) (cette incapacité est représentée par la croix), mais lorsqu'il y a du HF, il est transformé en estradiol natif (Es) qui peut alors être reconnu par le premier anticorps (AcS). Il entre alors en compétition avec le traceur (T) (estradiol marqué). La présence de HF peut donc être détectée. La quantité de traceur lié à l'anticorps est ici inversement proportionnelle à la quantité d'estradiol présente dans l'échantillon.

Dans ce mode de détection par compétition, le moyen permettant de détecter l'interaction entre le premier anticorps (AcS) et le composé organique non silylé, (ou le moyen permettant de détecter une interaction entre le premier anticorps et le composé organique silylé) peut comprendre un deuxième anticorps (AcC). Par exemple, ce deuxième anticorps peut être un anticorps de chèvre ou de lapin (AcC) dirigé contre l'anticorps de souris précité (AcS) (premier anticorps). Une représentation schématique de cette détection avec un deuxième anticorps a été ajoutée sur la figure 2 annexée. Ce deuxième anticorps (AcC) peut être fixé au fond d'un réceptacle (R) par exemple d'une plaque de microtitration.

Le dosage consiste donc dans cet exemple à effectuer une compétition entre le traceur et l'estradiol à doser vis-à-vis d'un nombre limité de molécules d'anticorps. En fin de réaction et après élimination du traceur non lié aux anticorps, le signal porté par le traceur lié aux anticorps peut être mesuré.

Lorsque le traceur comporte une enzyme, par exemple l'acétylcholine estérase (schématisé par le carré blanc sur la figure 2), le moyen de détection peut comprendre en outre un révélateur enzymatique. Dans l'exemple précité d'utilisation de l'acétylcholine estérase, le révélateur enzymatique peut être constitué d'un mélange d'acétylthiocholine et de dithionitrofluoro-benzène. Ce révélateur est transformé en produit de couleur jaune visible à l'oeil nu ou quantifiable par un spectrophotomètre à 414 nm. Ce révélateur est en fait utilisé pour appliquer la méthode de détection et de dosage colorimétrique d'Ellman pour la mise en oeuvre de la présente invention. Cette méthode est décrite par exemple dans le document référencé [43].

Selon une deuxième variante de ce deuxième mode de réalisation de la présente invention, la détection et/ou le dosage du fluor selon le procédé de l'invention est réalisé au moyen d'un immunodosage de type « non compétitif ». Par rapport au dosage compétitif, la difficulté de l'approche non compétitive est liée au composé organique choisi qui doit posséder deux propriétés chimiques: la possibilité d'être couplé à une phase solide et la possibilité d'être silylé. En outre, une fois couplé à la phase solide il doit pouvoir être désilylé dans des conditions aussi avantageuses qu'en phase liquide. Cette variante peut s'appliquer aux composés organiques hydroxylés précités. Par exemple elle peut s'appliquer à des dérivés d'estradiol tels que ceux précités, par exemple l'estradiol 3-carboxyméthyl éther, ou à des peptides, par exemple l'AcSDKP précité.

Dans cette variante, le composé silylé est couplé à une phase solide, par exemple un support, par exemple une plaque de microtitration telles que celles utilisées couramment en laboratoire. L'échantillon peut être mis en contact avec le composé silylé ainsi immobilisé sur la plaque, et la détection et/ou le dosage peut être effectué in situ.

L'immobilisation ou couplage du composé silylé sur le support peut se faire par toute technique appropriée connue de l'homme du métier pour fixer un des composés organiques silylé tels qu'ils sont définis ci-dessus sur un support tout en préservant la réactivité du composé silylé avec les ions fluorures. Tout dépend de la nature chimique du support et du composé organique silylé choisi pour la mise en oeuvre de la présente invention. Cette fixation peut se faire par exemple au moyen d'une liaison covalente. Le support peut être un support fonctionnalisé, c'est. à dire un support sur lequel ont été greffé des groupes chimiques facilitant la fixation du composé organique silylé. Il peut s'agir par exemple d'une plaque comportant des groupes aminés, par exemple une plaque Nunc-NH₂, ou d'une plaque activée avec de la polylysine, par exemple lorsque le composé organique silylé choisi est un dérivé d'estradiol tels que ceux précités, par exemple l'estradiol 3-carboxyméthyl éther, ou à un peptide, par exemple l'AcSDKP.

Après réaction avec les ions fluorure, s'ils sont présents dans l'échantillon, le composé silylé fixé sur le support se transforme en composé non silylé. La détection et/ou le dosage peut alors être réalisée par une technique d'immunodosage, par exemple en présence d'un premier anticorps reconnaissant spécifiquement le composé non silylé.

La révélation de la reconnaissance du composé silylé par le premier anticorps peut se faire par tout moyen connu de l'homme du métier, par exemple par marquage du premier anticorps, mais aussi au moyen d'un deuxième anticorps reconnaissant le premier anticorps et qui porte un marqueur (il constitue alors un traceur). Le marqueur peut être par exemple un de ceux habituellement utilisés par l'homme du métier, par exemple un signal enzymatique. Par exemple, le deuxième anticorps peut être un anticorps de chèvre ou de lapin (AcC) dirigé contre le premier anticorps qui peut être un anticorps de souris (AcS).

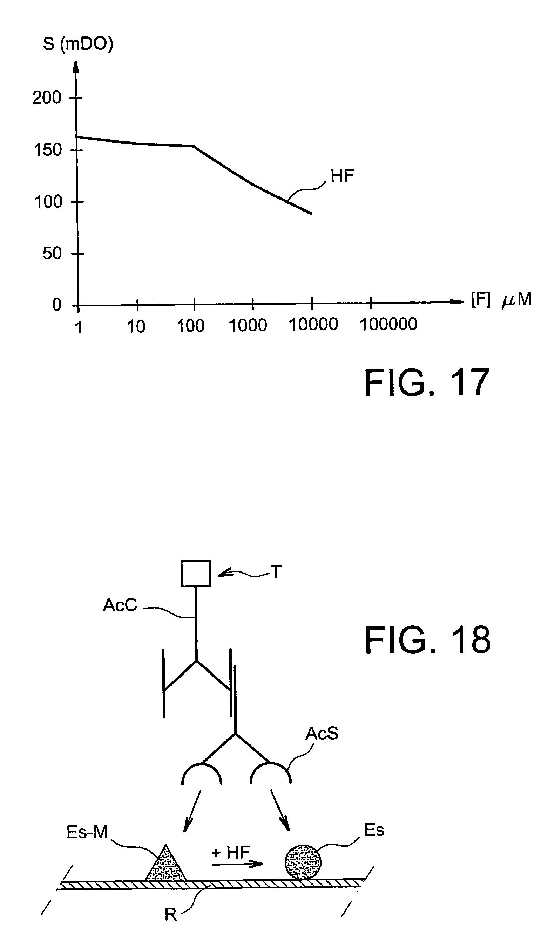

Un mode de détection « non compétitif » est représenté schématiquement sur la figure 18. Le composé organique utilisé sur cette figure est l'estradiol carboxyméthyl éther (EstCME). Un premier des anticorps utilisés est un anticorps anti-estradiol (par exemple de souris) référencé « AcS » (traceur). L'estradiol non silylé (non modifié) est référencé « Es » et l'estradiol silylé (modifié) « Es-M ». Un deuxième anticorps de chèvre ou de lapin (AcC) dirigé contre l'anticorps de souris (AcS) est ajouté, par exemple sur la surface d'une plaque de microtitration (R). L'anticorps AcC est fixé à un marqueur par exemple une enzyme pour former le traceur « T ». Cette figure montre que l'anticorps (AcS) est incapable de reconnaître l'estradiol modifié (Es-M) (cette incapacité est représentée par la croix), mais lorsqu'il y a du HF, Es-M est transformé en estradiol natif (Es) qui peut alors être reconnu par le premier anticorps (AcS). La présence de HF peut donc être détectée. La quantité de traceur (AcC + marqueur) lié à l'anticorps AcS est ici proportionnelle à la quantité d'estradiol désilylé présente dans l'échantillon.

Quelque soit le mode de réalisation de l'invention, des conditions opératoires avantageuses de mise en oeuvre du procédé de la présente invention ont été déterminées par les inventeurs. Ces conditions peuvent bien entendu être adaptées ou modifiées si nécessaire pour d'autres groupes silyl utilisés, d'autres composés organiques silylés, ou d'autres moyens de détection au sens de la présente invention. Ces conditions sont en particulier avantageuses pour le deuxième mode de réalisation de la présente invention.

Ainsi, l'étape de mise en contact du procédé de l'invention peut avantageusement être réalisée à une température de 54 à 64°C. En effet, des phénomènes de désilylation spontanée du composé organique silylé peuvent apparaître à des températures trop élevées. L'homme du métier saura adapter sans difficulté la température de mise en oeuvre du procédé de l'invention en fonction du composé organique et de la méthode de détection choisis.

De manière avantageuse également, la mise en contact du procédé de l'invention peut être réalisée à pH 4,5 à 6,5, de préférence à pH 5,5, par exemple dans un tampon phosphate à 50mM ou au moyen de tout tampon approprié. En effet, des phénomènes de désilylation spontanée du composé organique silylé peuvent apparaître à des pH trop acides. Là aussi, L'homme du métier saura adapter sans difficulté le pH de mise en oeuvre du procédé de l'invention en fonction du composé organique et de la méthode de détection choisis.

Selon l'invention, la concentration du composé organique silylé, par exemple tel que l'estradiol ou un autre composé de poids moléculaire équivalent, dans la solution de mesure peut être adaptée en fonction du poids moléculaire du composé organique et du moyen de détection et/ou dosage utilisé. Généralement, elle peut être de 1 à 2000 ng/ml lors de la mise en oeuvre du procédé de l'invention, par exemple de 2 à 500 ng/ml.

La présente invention se rapporte également à une trousse pour la mise en oeuvre du procédé de l'invention, ladite trousse comprenant les réactifs suivants : un composé organique silylé qui est désilylé lorsqu'il est en présence de fluor ou d'acide fluorhydrique ; et un moyen permettant de détecter l'apparition du composé organique désilylé ou la disparition du composé organique silylé en solution aqueuse.

Le composé organique silylé qui est désilylé lorsqu'il est en présence de fluor ou d'acide fluorhydrique est tel que défini dans la présente.

Le moyen permettant de détecter et/ou de doser l'apparition du composé organique désilylé ou la disparition du composé organique silylé en solution aqueuse dépend bien entendu du procédé de détection et/ou de dosage utilisé pour la mise en oeuvre du procédé de l'invention. Les procédés utilisables sont tels que définis ci-dessus. Le moyen de détection et/ou de dosage peut donc comprendre un ou plusieurs des éléments suivants: des indicateurs colorés, des marqueurs tels que ceux précités, des enzymes, des moyens de mesure tels que ceux précités, des anticorps nécessaires à la détection et/ou au dosage du composé organique silylé et/ou non silylé, etc.

Par exemple, la trousse peut comprendre des réactifs et anticorps nécessaires à la mise en oeuvre d'un immunodosage de type compétitif ou des réactifs et anticorps nécessaires à la mise en oeuvre d'un immunodosage de type non compétitif. La trousse peut comprendre un ou plusieurs anticorps, par exemple de souris, dirigé(s) contre le composé organique non silylé ou désilylé, ou contre le composé organique silylé. Elle peut comprendre en outre un ou plusieurs anticorps, par exemple de chèvre ou de lapin, dirigés contre les anticorps précités. Elle peut également comprendre un traceur, permettant de mettre en évidence la réaction immunologique mie en oeuvre.

Selon l'invention, la trousse peut en outre comprendre un support pour recevoir les réactifs, par exemple une barrette de polystyrène, dans lequel sont formés un ou plusieurs puits qui peuvent servir de réceptacle(s) pour l'étape de mise en contact et/ou de détection et/ou dosage du procédé de l'invention. Le dosage et/ou la détection peut en effet aisément être réalisé dans des puits de plaques de microtitration. Sur un tel support, par exemple la barrette précitée, les puits peuvent être recouverts d'anticorps , par exemple de chèvre ou de lapin, dirigés contre les anticorps de souris anti-estradiol. L'estradiol silylé peut également être fixé au fond des puits.

Les inventeurs ont, grâce à la présente invention, réussi à développer un test pratique et rapide, permettant de détecter HF à des concentrations qui peuvent être de l'ordre de 0,001 µg/ml en solution.

La sensibilité du procédé de l'invention permet avantageusement d'envisager toutes les applications industrielles et notamment la mesure de l'acide fluorhydrique atmosphérique qui est la plus utile. Dans ce cas, la détection de HF peut aisément être réalisée pour 1x10⁻² l de HF / 10⁶ l d'air (10 ppb). Ce procédé possède donc des performances supérieures à celles des dosages de terrain de l'art antérieur qui étaient limités à des détections de HF de l'ordre de quelques centaines de ppb.

La présente invention permet en général d'obtenir un résultat en 1/2 à 4 heures environ, suivant la nature de l'échantillon et des moyens de détection mis en oeuvre. Même si certaines durées sont plus grandes que celles de certains procédés de l'art antérieur, le procédé de l'invention est de manière générale plus sensible.

Le procédé de l'invention s'applique avantageusement à tous les échantillons contenant de l'acide fluorhydrique ou des ions fluorures : prélèvement atmosphérique, aliments, végétaux, milieux biologiques. Dans chaque cas, les conditions de prélèvement et de préparation de l'échantillon sont communes à toutes autres méthodes de dosage de l'acide fluorhydrique ou des ions fluorures.

Un autre avantage de la présente invention est qu'elle s'effectue d'une façon simple. Par exemple, la technologie peut être identique à celle des immunodosages : utilisation de réactifs prêts à l'emploi, manipulation de distributions simples, encombrement réduit, transport et délocalisation faciles, possibilité de travailler sans apport d'énergie, lecture à l'oeil nu ou par colorimétrie au moyen d'un simple spectrophotomètre de poche.

D'autres applications et avantages de la présente invention apparaîtront encore à la lecture de la description qui suit donnée à titre illustratif et non limitatif en référence aux figures annexées.

### Brève description des figures

- La figure 1 est une représentation schématique mettant en évidence la partie la molécule d'estradiol (Es) reconnue par son anticorps (Ac). La fixation d'un atome de Si sur le -OH de la partie de la molécule d'estradiol reconnue par l'anticorps empêche l'anticorps de reconnaître cette molécule.
- La figure 2 est un schéma général de la détection de HF au moyen du procédé de l'invention suivant le premier mode de réalisation où la détection est réalisée par compétition.
- La figure 3 est une représentation schématique d'un immunodosage par compétition (à gauche) et d'une mesure de signal obtenu avec un tel dosage (à droite). Sur cette figure, S(%) représente le signal émis par le traceur (T) lié à l'anticorps (Ac); et [Es](M) représente la concentration molaire (mol/l) de l'estradiol natif.
- La figure 4 est un graphique montrant l'effet de différents acides organiques sur la désilylation de l'estradiol-triméthylsilyl obtenu avec du BTSFA (B/Bo est une mesure du signal sous forme d'un rapport entre le signal en présence de HF (B (pour "bound" en anglais)) et le signal en l'absence de HF (Bo) (pour "unbound" en anglais)) dans un test immunologique suivant le protocole de l'exemple 2 ci-dessous. [H⁺](M) représente la concentration molaire en acide pour chaque acide identifié sur ce graphique.
- La figure 5 est un graphique montrant une étude des conditions de désilylation de l'estradiol : température, et pH du tampon phosphate (P) 50mM (comme sur la figure 4, on mesure le signal B/Bo).
- La figure 6 est un graphique montrant l'effet de différents acides sur la désilylation de l'estradiol silylé en tampon phosphate 50mM, pH 5,5. [H⁺](M) représente la concentration molaire en acide pour chaque acide identifié sur ce graphique. Les données sont similaires avec l'acide chlorhydrique, nitrique ou sulfurique (comme sur la figure 4, on mesure le signal B/Bo).
- La figure 7 est un graphique montrant la spécificité de détection de HF par le procédé de l'invention comparée à d'autres nucléophiles et halogènes. [P](M) représente la concentration molaire en halogène ou autre nucléophiles pour chaque halogène identifié sur ce graphique (comme sur la figure 4, on mesure le signal B/Bo).
- La figure 8 est un graphique montrant la spécificité de détection de HF par le procédé de l'invention face à différents sels. [X⁺F⁻](M) représente la concentration molaire en sel de fluor pour chaque sel identifié sur ce graphique (comme sur la figure 4, on mesure le signal B/Bo).
- La figure 9 est un graphique montrant la corrélation entre la concentration en fluor [F] (en pmol/1) détectée (d) par le procédé de l'invention et théorique ([F]ₜₕ) dans des échantillons d'atmosphère contaminée par du fluor.

Les figures 10 à 14 sont des représentations graphiques de résultats expérimentaux de reconnaissance d'estradiol non silylé par des anticorps spécifiques suivant des protocoles de dosage immunologique de type non compétitif. Sur ces figures :

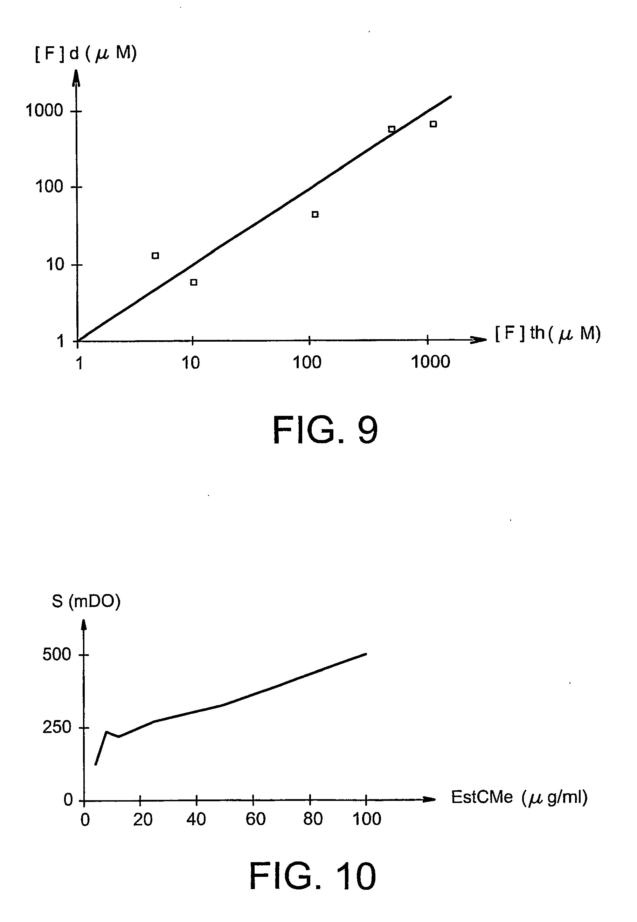

- La figure 10 représente le signal (S) (mDO) de couplage de l'estradiol carboxyméthyl éther (EstCME) sur des plaques de microtitration possédant des groupes aminés (plaques Nunc aminés) en fonction de la concentration de l'estradiol (en µg/ml).

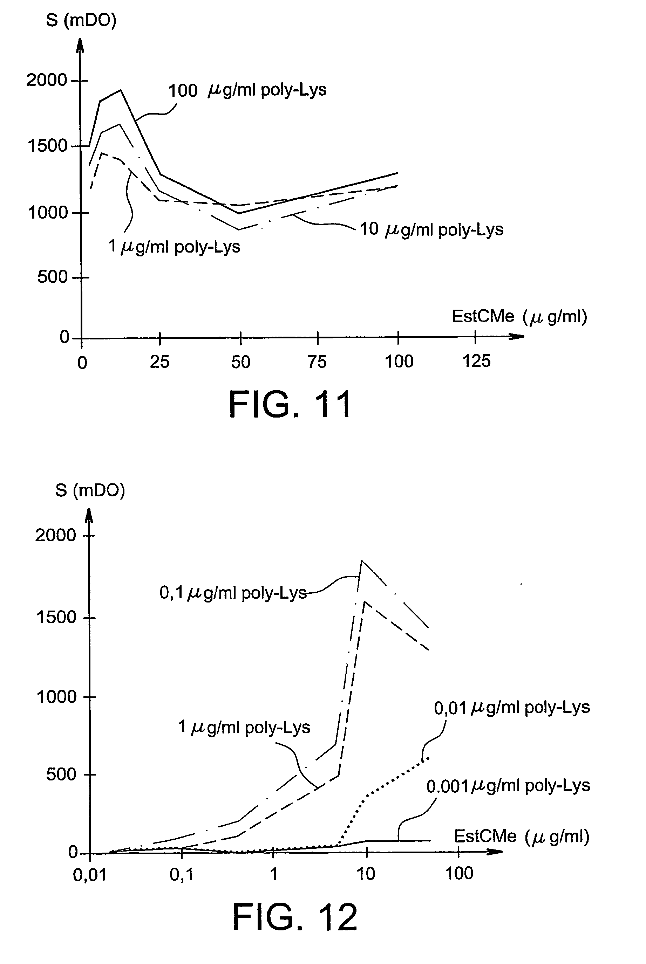

- Les figures 11 et 12 représentent le signal (S) (mDO) (Absorbance mesurée à 414nm) de couplage de l'estradiol carboxyméthyl éther sur des plaques de microtitration activées par de la polylysine (poly-Lys) en fonction de la concentration en polylysine (en µg/ml).

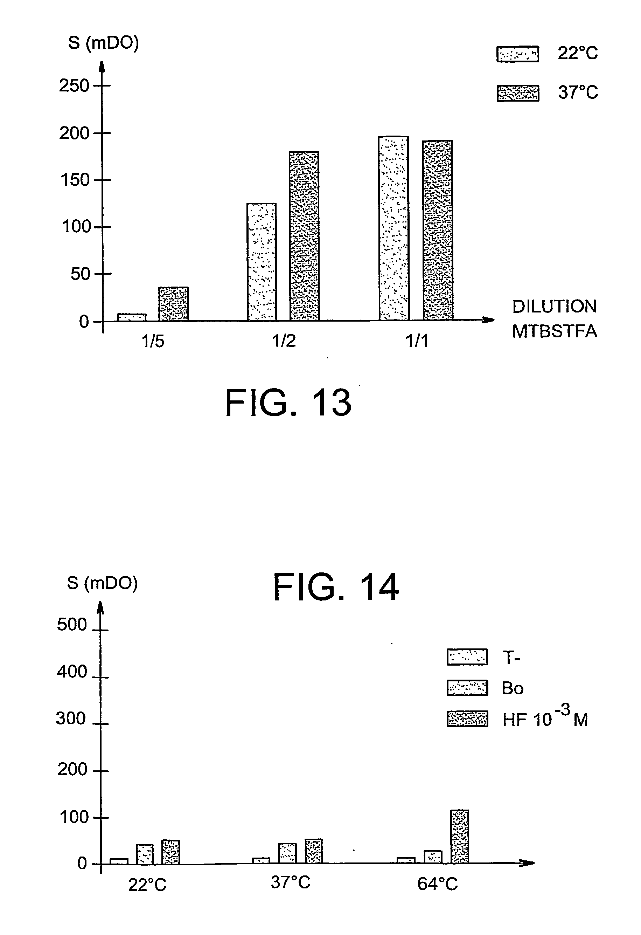

- La figure 13 représente un test de silylation de l'estradiol carboxyméthyl éther par le MTSBTFA : l'efficacité de la silylation est mesurée par l'augmentation du signal (S) (mDO), en fonction de la dilution de MTSBTFA (1/5; ½; 1/1), et de la température en degrés Celsius (°C).
- La figure 14 représente un test du format immunométrique après silylation de l'estradiol carboxyméthyl éther : le signal (S) (mDO) est mesuré en fonction de la présence ou l'absence de HF. Sur cette figure, Bo représente le signal en l'absence d'HF (témoin).

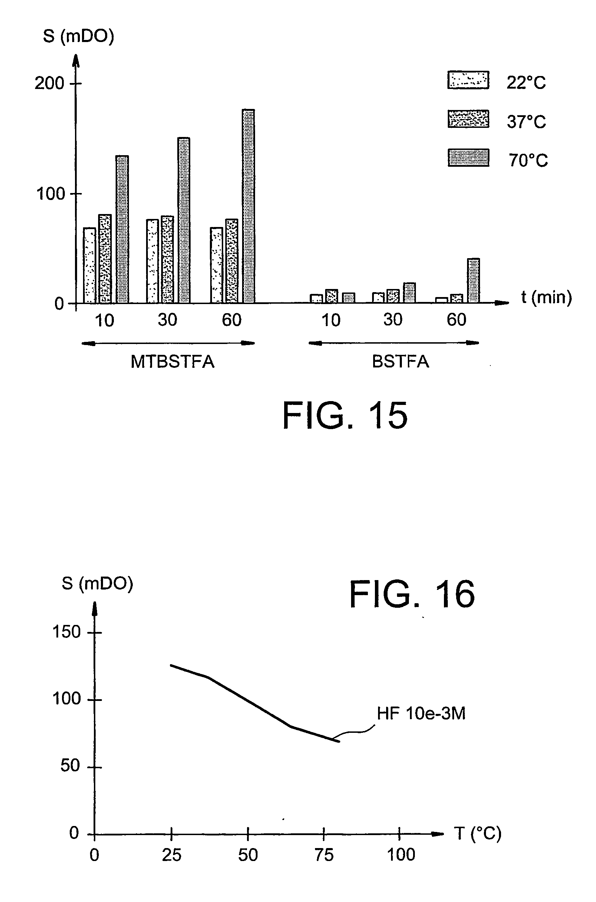

- La figure 15 représente, sous forme de graphique, des résultats expérimentaux de mises en oeuvre du procédé de l'invention avec un composé organique silylé qui est un tétrapeptide AcSDKP : mesure du signal (S(mDO)) à 414nm. Deux séries d'essais ont été réalisés : une série avec le tétrapeptide silylé avec un premier réactif de silylation (MTSBTFA), et une série avec le tétrapeptide silylé avec un deuxième réactif de silylation (BSTFA). Pour chaque série, trois essais ont été réalisés: à 22°C, à 37°C et à 70°C, et des mesures ont été effectuée à 10, 30 et 60 minutes à chaque fois.
- La figure 16 est une représentation graphique de résultats expérimentaux obtenus dans une étude de l'efficacité de détection du fluor par le procédé de la présente invention en fonction de la température de désilylation (T(°C)) : mesure du signal (S(mDO)) à 414nm. Le composé silylé utilisé est celui de la figure 15 obtenu avec le MTBSTFA.
- La figure 17 représente, sous forme de graphique, des résultats expérimentaux de mises en oeuvre du procédé de l'invention avec un composé organique silylé qui est un dérivé de l'estradiol : mesure du signal (S(mDO)) à 414nm en fonction de la concentration en fluor ([F] en micromoles/l (µM).
- La figure 18 représente schématiquement le mécanisme d'une détection immunologique non compétitive telle qu'elle peut être utilisée dans l'étape de détection du procédé de l'invention.

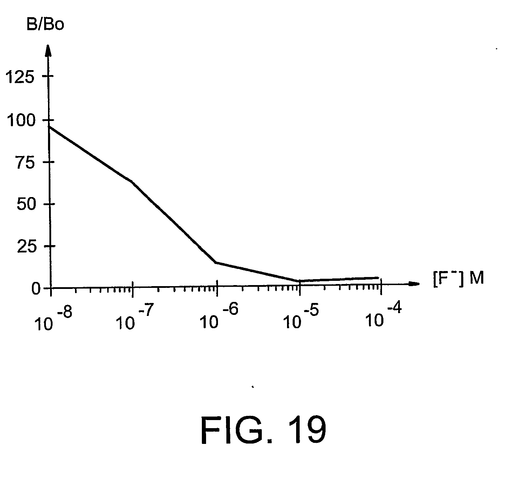

- La figure 19 est un graphique montrant les résultats de détection du fluor selon le procédé de l'invention, en présence de diméthylsulfoxide (DMSO) : B/Bo est une mesure du signal sous forme d'un rapport entre le signal en présence de HF (B) et le signal en l'absence de HF (Bo) et [F⁻] (M) la concentration molaire en ions fluorures.

### Exemples

Les différents aspects du procédé de l'invention ont été étudiés ci-après: mise en place du dosage immunologique de l'estradiol, tests des conditions de modification de l'estradiol par silylation, vérification des composés organiques silylés par spectrométrie de masse, test de réaction des composés silylés avec le HF, optimisation de la nature chimique des dérivés silylés, optimisation des concentrations de réactifs, sensibilisation du dosage, optimisation de la spécificité du dosage par rapport aux acides, test d'applicabilité du dosage. Les principaux résultats de ces études sont exposés ci-dessous.

Les tests ont été effectués sur des échantillons d'acide fluorhydrique atmosphérique (simulation au laboratoire) et sur des échantillons d'eau contenant des ions fluorures.

Afin de déterminer les performances du procédé de l'invention, les inventeurs ont défini ses paramètres de sensibilité. La sensibilité représente la quantité de HF nécessaire pour engendrer un signal statistiquement différent du signal obtenu en l'absence de HF. Suivant le procédé de l'invention, la quantité d'estradiol natif (non silylé) présent dans l'échantillon à doser dépend de la quantité de fluor qui va permettre la transformation d'estradiol silylé non détectable en estradiol non silylé détectable. La sensibilité a donc été définie, pour le dosage de HF, comme la quantité de fluor nécessaire qui, mis en présence de l'estradiol-diméthyl-terbutyl, est capable d'induire une diminution significative de la liaison entre l'anticorps et le traceur enzymatique.

### Exemple 1 : application du procédé de l'invention à la détection et au dosage du fluor par immunodosage compétitif

Dans cet exemple, les composés organiques choisis sont des composés dérivés du β17-estradiol.

### 1) Préparation de l'anticorps Anti-estradiol

L'anticorps utilisé dans le dosage a été produit notamment partir de composés dérivés du β17-estradiol et couplés à l'albumine bovine dans le but d'obtenir des anticorps chez la souris.

La technique utilisée est celle décrite dans la référence [39].

### 2) Préparation d'un anticorps anticorps de souris de l'anticorps Anti-estradiol

Des anticorps de chèvre anti-anticorps de souris commercialisés par la société Immunotech (Lumigny) sont utilisés pour recouvrir (dilution d'utilisation : 5 µg/ml) des plaques de microtitration (Nunc) de 96 puits en polystyrène. Après recouvrement, les plaques sont conservées dans un tampon contenant de l'albumine à 0,5% et à 4°C.

La fixation sur la plaque se fait toute seule par adsorption. L'incubation doit être suffisante pour que la protéine puisse s'accrocher. Cette préparation fait partie des connaissances générales de l'homme du métier.

### 3) Préparation du traceur enzymatique

Le traceur enzymatique a été obtenu par couplage de l'estradiol avec l'acétylcholinestérase (AChE). Des groupements thiolés sont introduits sur l'estradiol grâce au N-succinimidyl S-acétylthioacétate. L'estradiol ainsi modifié est couplé à l'AChE sur laquelle ont été introduits des groupes maléimides réagissant avec les groupements thiolés de l'estradiol. Le traceur a été purifié par gel de filtration puis conservé sous forme d'aliquote à -20°C.

Les documents référencés [44] et [45] décrivent les techniques et les traceurs enzymatiques utilisés pour cette préparation, et utilisables dans la présente invention de manière générale.

### 4) Produits et autres réactifs

L'estradiol a été obtenu chez Sigma Aldrich. Les agents silylants ont été obtenus auprès de la société Perbio.

L'acide fluorhydrique provient de la société Merck-Eurolab.

Tous les réactifs - traceur, anticorps, estradiol et échantillons - ont été utilisés dilués dans un tampon phosphate 0,05 M, pH 7 comprenant de l'azide (0,01% p/v) (% p/v = rapport poids en g sur volume de 100 ml) (soit ici 0,01 g/100ml) de l'albumine bovine (0,5% p/v) et du NaCl (0,9% p/v).

### 5) Optimisation de la concentration d'estradiol silylé utilisée

La concentration d'estradiol silylé mis en contact avec l'échantillon contenant le HF a été optimisée pour permettre par la suite une détection de l'estradiol désilylé et donc du fluor.

En effet, si la concentration en estradiol silylé utilisée est très faible et même si le tout est transformé en estradiol, la faible concentration ne permet pas toujours une grande précision de détection.

Un certain nombre de tests ont été effectués afin de déterminer la concentration optimum d'estradiol modifié à utiliser lors de la désilylation dans le procédé de l'invention. Cette concentration varie bien entendu en fonction des réactifs utilisés et des conditions opératoires.

Dans les exemples présentés ici, elle est de préférence de 200 ng/ml.

On peut estimer que cette concentration sera en général de 1 à 2000ng/ml pour l'estradiol et ses dérivés, de préférence de 2 à 500ng/ml, ces fourchettes n'étant données qu'à titre indicatif, en particulier pour les conditions de l'exemple.

### 6) Silylation de l'estradiol

Un volume d'estradiol (2 mg/ml) est mélangé à 4 volumes de MTBSTFA et est incubé à température ambiante pendant 30 minutes à 1 heure.

Les agents silylants utilisés sont BTSFA ou le MTBSTFA. Les produits susceptibles d'être obtenus sont représentés ci-dessus.

Une vérification par chromatographie liquide et spectrométrie de masse a montré que la silylation de l'estradiol a été réalisée.

L'estradiol une fois silylé est dilué d'un facteur 1000 dans du diméthylformamide (DMF), ou alors dans du diméthylsulfoxide (DMSO), à 100% avant utilisation.

### 7) Désilylation de l'estradiol

A 50 µl d'estradiol silylé sont ajoutés 2,5 µl de tampon phosphate 1 M (pH = 5,5) et 47,5 µl d'échantillon ou d'une solution de HF de concentration connue (gamme d'étalonnage).

Le mélange est agité puis laissé à 1 heure à 55-66°C dans un bain-marie à sec.

La réaction de désilylation peut être stoppée par dilution du mélange dans un tampon de dosage (dilution d'un facteur 40) qui est du tampon phosphate 0,05 M, pH 7 comprenant de l'azide (0,01% p/v) (% p/v = rapport poids en g sur volume de 100 ml) (soit ici 0,01 g/100ml) de l'albumine bovine (0,5% p/v) et du NaCl (0,9% p/v) .

### 8) Révélateur enzymatique : Mesure de l'activité enzymatique de l'acétylcholinestérase (réaction d'Ellman)

La méthode utilise un pseudo substrat : l'acétylthiocholine (AcTCh) qui est hydrolysé à la même vitesse que l'acétylcholine. Cette hydrolyse conduit à la formation de thiocholine (TCh) qui a la faculté de réduire le dithionitrobenzène (DTNB). Le DTNB réduit absorbe fortement dans le visible (ε_{M 412 nm} = 13600 mol⁻¹.cm⁻¹.l) en produisant une coloration jaune.

L'AcTCh est utilisée à une concentration pour laquelle l'activité de l'AChE est maximale en tenant compte de l'inhibition par excès de substrat. De plus, le DTNB est mis en défaut par rapport à l'AcTCh de façon à éviter des mesures colorimétriques après transformation de tout le substrat. Par contre, le DTNB est mis en excès par rapport à la quantité de TCh produite de façon à ce que l'hydrolyse d'une molécule d'AcTCh conduise à la formation d'une seule molécule de DTNB réduit.

L'homme du métier sait mettre en oeuvre la réaction d'Ellman et peut sans difficulté trouver les conditions opératoires optimales pour mettre en oeuvre l'invention à partir des informations indiquées ici.

### 9) Immunodosage de l'estradiol natif quantification de HF

Les extraits d'estradiol obtenus précédemment sont dilués au 1/40 et sont répartis sur les plaques de dosage en présence du traceur et des anticorps anti-estradiol. Le volume réactionnel est de 200 µl.

La réaction immunologique dure une heure à température ambiante.

Les plaques de dosage sont lavées pour éliminer le traceur non fixé et 0,2 ml de révélateurs sont ajoutés.

Après 1 h environ, la couleur jaune apparaît et des mesures de densité optique sont effectuées par un spectrophotomètre.

### 10) Interprétation des résultats

A partir d'une gamme d'étalonnage du HF, la concentration du fluor contenu dans les échantillons a pu être déterminée dans les essais réalisés, démontrant ainsi la faisabilité du procédé de l'invention.

### Exemple 2 : étude de la différence de spécificité de détection du HF pour l'estradiol modifié par MTBSTFA et pour l'estradiol modifié avec BTSFA

Dans toutes les manipulations décrites par la suite, l'estradiol silylé était utilisé lors de la désilylation à une concentration fixée pour tous les échantillons d'une même expérience. Cette concentration était de 200 ng/ml.

Pour les autres réactifs, les concentrations étaient les suivantes :
- Anticorps anti-estradiol de souris : 5µg/ml ;
- Acétylthiocholine : 7x10⁻⁴ M ;
- 5-5'-dithiobis-nitrobenzoate : 7x10⁻⁴ M ; et
- Estradiol couplé à l'acétylthiocholine : environ 1 ng/ml.

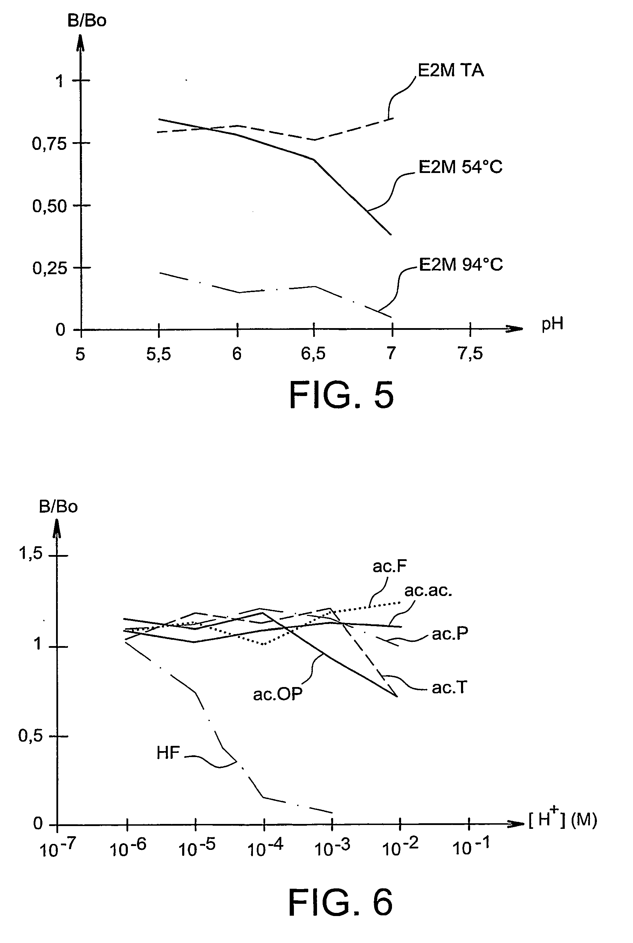

Les tests ont été réalisés dans des puits de 300µl sur des barrettes de polystyrène de la manière exposée dans l'exemple 1.
A) L'estradiol silylé avec le BTSFA (qui transfert un groupement triméthylsilyl) est clivé (réaction de désilylation) pour retrouver sa forme native avec le HF. Un tel clivage peut aussi être observé avec d'autres acides : acide chlorhydrique (HCl), acide nitrique (HNO₃), acide sulfurique (H₂SO₄) et acide phosphorique (H₂PO₄). Les résultats sont présentés sur la figure 4 annexée.
   Il n'y a donc pas toujours de spécificité du fluor dans la réaction de désilylation en utilisant ce groupement silyl. Il est donc plutôt adapté à une détection du HF lorsque celui-ci est seul, ou alors à une détection de HF lorsque les autres acides ne sont pas présents.
B) L'estradiol silylé avec le MTBSTFA (qui transfère un groupement silyl plus hydrophobe qu'avec BTSFA), bien que montrant une solubilité du produit obtenu en milieu aqueux moins grande, est clivé (réaction de désilylation) de manière spécifique par HF pour retrouver sa forme native. En effet, comme le montre la figure 6 annexée, réalisée dans les mêmes conditions, les autres acides testés ne clivent pas l'estradiol ainsi modifié (voir exemple 3 ci-dessous).

Les inventeurs ont donc alors choisi le MTBSTFA pour silyler l'estradiol, dans les exemples ci-dessous.

### Exemple 3 : Recherche de tampons appropriés pour la réaction de désilylation avec le fluor dans le procédé de l'invention

Certaines entités acides ayant aussi un effet sur la désilylation de l'estradiol modifié par le MTBSTFA (voir exemple 2), les inventeurs ont décidé de rechercher des solutions tampons permettant d'accentuer l'effet spécifique du fluor sur la réaction de désilylation et d'inhiber l'effet non spécifique dû au caractère acide des autres composés qui peuvent être présents.

Les bases telles que la soude (NaOH) et la potasse (KOH) à fortes concentrations (nécessaires pour tamponner les fortes concentrations d'acide) provoquent aussi une désilylation de l'estradiol en l'absence d'acide (données non représentées). Elles ne sont donc pas utilisables.

Des solutions de tampon phosphate (KH₂PO₄/K₂HPO₄) à 50 mM en ion phosphate ont également été testées. Elles ont montré de bons résultats. Le pH de ces solutions a été étudié afin qu'une désilylation spontanée de l'estradiol silylé n'intervienne pas à cause de l'acidité de celles-ci.

Le procédé de l'invention peut donc avantageusement être réalisé en milieu tamponné, par exemple lorsque l'échantillon est susceptible de comprendre d'autres acides ou bases qui peuvent provoquer la désilylation spontanée du composé organique et donc réduire la sensibilité de détection et/ou de dosage du procédé de l'invention.

Par ailleurs, les inventeurs ont constaté que dans certaines conditions, une température de désilylation trop élevée pouvait engendrer une désilylation spontanée de l'estradiol modifié (données non présentées). C'est pourquoi chacun des différents agents tamponnant a été étudié à différentes températures.

La figure 5 annexée montre les résultats obtenus. Sur cette figure, « E2M » représente l'estradioldiméthylsilylterbutyl et « T.A. » représente la température ambiante.

Il apparaît une désilylation spontanée de l'estradiol silylé à 94°C, alors qu'à 54°C, il n'y en a pas pour des solutions de tampon phosphate à pH 5,5 ou 6.

En conclusion, les conditions préférées de désilylation d'estradiol silylé ont été fixées de la manière suivante: température comprise entre 54 et 64°C, dans un tampon phosphate 50 mM à pH 5,5. Bien entendu, ces conditions ont été déterminées pour l'estradiol et et le groupement silyl précité, et dans les conditions opératoires précitées.

D'autres conditions pourront aisément être déterminées par l'homme du métier en fonction du composé organique silylé choisi, de l'échantillon, et des conditions dans lesquelles le procédé de l'invention est mis en oeuvre à partir des informations fournies dans la présente.

### Exemple 4 _{:} Effet de différents acides sur la mise en oeuvre du procédé de l'invention

Dans les conditions .précitées de l'exemple 3, les effets de différents autres acides sur la désilylation ont été testés.

Les résultats de ces tests sont représentés sur la figure 6. Sur cette figure, « ac.F » représente l'acide formique ; « ac.ac » l'acide acétique ; « ac.P » l'acide phosphorique ; « ac.OP » l'acide ortho-phosphorique » et « ac.T » l'acide trifluoroacétique.

Ces résultats montrent clairement que l'effet des autres acides que HF sur l'estradiol silylé par MTBSTFA est quasiment nul.

Le groupement silyl choisi joue donc un rôle dans la spécificité de détection du fluor. Ce fait est à considérer lorsque d'autres éléments chimiques que le fluor se trouvent dans l'échantillon testé, surtout si ces éléments peuvent provoquer la désilylation du composé organique utilisé.

### Exemple 5 : Spécificité du fluor par rapport à d'autres halogènes et nucléophiles

Le fluor est un halogène et il a par ailleurs un caractère nucléophile. Afin de tester la spécificité du fluor dans la réaction de désilylation de l'estradiol, l'effet d'autres halogènes comme Bu₄NBr, Bu₄NCl, Bu₄NI et KI, et de nucléophiles comme le 4-nitrophénol et le 4-nitroimidazole a été étudié dans les conditions opératoires des exemples précédents, l'estradiol étant silylé par MTBSTFA.

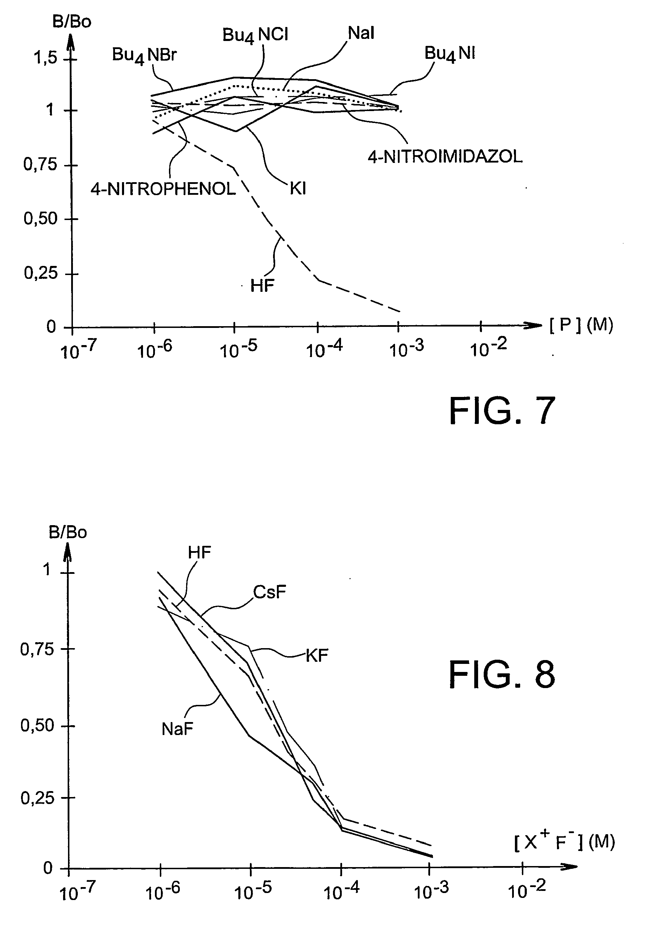

La figure 7 annexée représente les résultats expérimentaux obtenus dans cet exemple, et montre l'effet spécifique du fluor face aux autres produits.

Il apparaît clairement que le fluor est très spécifique dans la réaction de désilylation de l'estradiol par rapport aux autres produits testés.

### Exemple 6 _{:} Clivage par HF et par des ions du fluor

Dans cet exemple, différents sels de fluor ont été mis en présence d'estradiol silylé par MTBSTFA dans les conditions expérimentales des exemples précédents.

La figure 8 annexée regroupe les résultats obtenus. Sur cette figure, les symboles chimiques usuels ont été utilisés pour identifier les différents sels du fluor testés.

Les ions du fluor se comportent de manière similaire au HF pour cliver l'estradiol silylé et confirme la spécificité du fluor sur la réaction.

### Exemple 7 : Performances analytiques du procédé de l'invention et effet de l'addition d'un solvant organique

Dans les conditions décrites dans les exemples ci-dessus, la sensibilité du dosage du HF est d'environ 5 x 10⁻⁵ M (soit 1 µg/ml) pour le HF dilué en milieu aqueux comme le montrent les figures 7 et 8.

Au cours de travaux complémentaires, les inventeurs ont noté que la sensibilité du procédé de l'invention peut encore être augmentée en ajoutant à la solution aqueuse de désilylation un solvant organique miscible à l'eau.

L'expérience a été réalisée avec du diméthylformamide (voir silylation de l'estradiol) (DMF) et du diméthylsulfoxide (DMSO).

La sensibilité peut être améliorée d'un facteur 500 (environ 10⁻⁷ M, soit 0,001 µg/ml) en utilisant le diméthylsulfoxide (DMSO) comme solvant pour mettre en oeuvre la désilyation suivant le procédé de l'invention.

La figure 19 représente la courbe dose-réponse pour les ions fluorures pour une réaction effectuée en présence de 95% en volume de dimethylsulfoxide par rapport au volume total de solution aqueuse de mise en contact. Ces résultats sont à comparer avec ceux des exemples précédents.

L'addition du solvant organique dans ladite solution aqueuse de mise en contact augmente donc de façon importante la sensibilité de détection et/ou de dosage du fluor par le procédé de l'invention.

### Exemple 8: Utilisation du procédé de l'invention dans lequel le composé de l'invention est un dérivé de l'estradiol

Dans cet exemple, le composé organique utilisé pour mettre en oeuvre le procédé de l'invention est un dérivé O-méthyl de l'estradiol (E2OCH₃) : le 17-béta-estradiol 3-méthyl éther de formule :

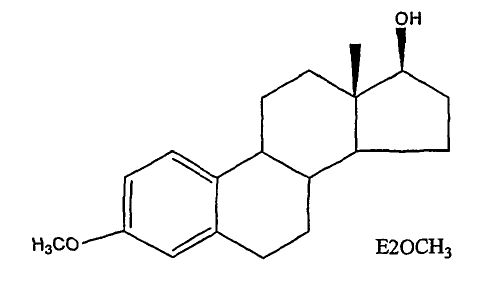

Les conditions opératoires utilisées sont celles exposées dans les exemples 1 et 2 ci-dessus.

Les résultats expérimentaux obtenus sont reportés sur la figure 17 annexée. On observe bien la désilylation de E2OCH₃ en présence de fluor.

L'utilisation d'un dérivé de l'estradiol dans le procédé de l'invention est donc probante.

### Exemple 9: Utilisation du procédé de l'invention dans lequel le composé organique est un peptide possédant des fonctions hydroxyles

Dans cet exemple, le composé organique est un peptide. Il s'agit d'un tétrapeptide Ser-Asp-Lys-Pro acétylé (AcSDKP) de formule:

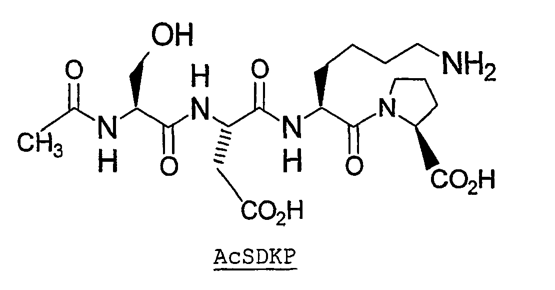

L'AcSDKP est un peptide disposant de groupements hydroxylés lui permettant avantageusement à la fois d'être silylé et d'être fixé sur une phase solide par ses groupes carboxylés ou aminés.

Dans un premier temps les réactions de silylation de ce peptide ont été étudiées, et dans un deuxième temps, l'utilisation de ce composé silylé dans le procédé de l'invention.

L'AcSDKP (1 mg/ml) a été silylé avec du MTBSTFA ou du BSTFA non dilué pendant 10, 30 ou 60 minutes à 22°C, 37 ou 60°C. Après réaction, l'AcSDKP silylé a été détecté par un dosage compétitif spécifique de l'AcSDKP au moyen de la technique décrite dans le document [5], en utilisant des anticorps polyclonaux de lapin. Dans ce cas une augmentation de signal indique une perte d'immunoréactivité et donc une efficacité de la silylation.

Comme le montre la figure 15 annexée sur laquelle sont reportés les résultats expérimentaux obtenus, le MTSBTFA à 60°C permet de diminuer la réactivité de l'AcSDKP et donc laisse conclure à une silylation du composé.

Par la suite des tests de désilylation de ce peptide silylé en présence de HF, conformément à la présente invention, ont été entrepris. La figure 16 annexée regroupe les résultats obtenus. On observe une désilylation du peptide avec le HF. En effet, la perte du groupement silylé conduit à une augmentation d'immunoréactivité et donc à un signal plus faible. Comme l'indique la figure, le signal diminue en présence de HF.

Le procédé de l'invention peut donc également être mis en oeuvre avec un peptide hydroxylé en tant que composé organique.

### Exemple 10 : Détection et dosage de fluor en solution

Afin de vérifier la validité du procédé de l'invention, les inventeurs ont testé différentes eaux minérales dont la concentration en fluor est connue.

Le protocole utilisé est celui de l'exemple 1, le composé organique choisi est l'estradiol silylé avec du MTBSTFA (estradiol-diméthyl-terbutyl).

Le tableau I ci-dessous regroupe les résultats obtenus dans cet exemple. Il montre que dans tous les cas la présence de fluor a été détectée.

La corrélation entre la concentration théorique et retrouvée semble correcte pour les eaux les plus concentrées. Il est possible que la nature du fluor présente dans l'échantillon soit la conséquence des quelques variations observées.

**Tableau I**

| **Dosage de fluorures dans des eaux minérales** | | | | | | |
|---|---|---|---|---|---|---|
| **Eaux** | **[fluorures]** | | **[extraits secs]** mg/ml | **pH** | **[F] Détectée** µM | **% de F retrouvée** |
| | mg/ml | µM | | | | |
| **Vichy St Yorre** | 9 | 470 | 4774 | 6,6 | 780 ± 271 | 170 ± 60 |
| **San-Pellegrino** | 0,61 | 32 | 1074 | 7,5 | 151 | 470 |
| **Badoit** | 1 | 52 | 1200 | 6 | 28,5 ± 12 | 50 ± 20 |
| **Vichy Célestin** | 6 | 135 | 3325 | 6,8 | 305 ± 81 | 97 ± 26 |
| **Quézac** | 2,1 | 100 | - | - | 53 | 53 |

### Exemple 11 : détection et dosage de fluor à partir de milieux gazeux

Le HF à doser est souvent sous forme gazeuse et il faut le faire passer en solution aqueuse pour le doser. N'ayant pas d'atmosphère contaminée par le HF, les inventeurs ont artificiellement recréé de tels échantillons, en faisant évaporer une solution de HF de concentration connue, dans un récipient fermé. Une fois le HF évaporé, l'atmosphère a été pompée et mise à buller dans un tampon de recueil (DMF).

Avec un système permettant de filtrer 100 litres d'air dont la concentration de HF est de 1,2 x 10⁻² l / 10⁶ l d'air (12 ppb), une concentration de HF à 10⁻⁵ M en solution (sous réserve d'un rendement de 100%) est obtenue.

Le protocole de détection du fluor utilisé est celui décrit dans l'exemple 1. Le composé organique choisi est est l'estradiol silylé avec du MTBSTFA (estradiol-diméthyl-terbutyl).

Les résultats présentés sur la figure 9 montrent que dans tous les cas, le fluor est détecté. La corrélation entre les concentrations théoriques et détectées de fluor est assez bonne.

Le procédé de détection de HF dans l'air selon l'invention permet donc aisément de détecter HF à des concentrations de l'ordre de 10⁻² l de HF / 10⁶ l d'air (10 ppb).

### Exemple 12 : application du procédé de l'invention à la détection et au dosage du fluor par immunodosage non compétitif

### 1) Stratégie générale

Le but est d'utiliser les résultats précédemment acquis, à savoir la possibilité de modifier la reconnaissance immunologique d'un composé silylé par l'action du HF.

Par rapport au précédent format de dosage (par compétition), l'approche immunométrique consiste à immobiliser le composé silylé de façon covalente sur une surface solide. Sa transformation par le HF conduit à une forme immunoréactive et donc à l'apparition d'un signal contrairement au dosage compétitif où il y a disparition du signal.

Ce format permet souvent une meilleure sensibilité probablement du fait d'une thermodynamique favorable liée aux réactifs qui sont en excès et une lecture plus facile sur la base qu'il est plus facile de voir ou mesurer l'apparition d'un signal que sa disparition.

### 2) Réactifs et protocole

L'anticorps utilisé est celui décrit dans la référence [39].

La biotinylation des anticorps de lapin et le marquage de la streptavidine ou des anticorps à l'acétylcholinestérase ont été réalisés au laboratoire par les techniques habituelles connues de l'homme du métier.

L'ensemble des produits et réactifs chimiques a été obtenu chez Sigma ou Merck. Les composés obtenus proviennent de chez Sigma ou Steraloids (pour les dérivés de l'estradiol).

Le tampon de dosage est un tampon phosphate 0,05 M, pH 7,4 comprenant de l'azide (0,01%), de l'albumine bovine (0,5%) et du NaCl (0,9%).

Le révélateur enzymatique est constitué d'un mélange d'acétylthiocholine et de dinitro-fluorobenzène. Après réaction avec le traceur, il produit une couleur jaune visible à l'oeil nu ou quantifiable par un spectrophotomètre à 414 nm.

Les plaques utilisées pour la réalisation des expériences, en particulier pour la fixation du dérivé de l'estradiol, proviennent de chez Nunc (Rochester, USA).

Les agents silylants utilisés sont le BSTFA et le MTBSTFA.

L'estradiol utilisé est indiqué ci-dessous. L'intérêt de ce composé est qu'il permet d'utiliser les anticorps pour la détection et le principe de base précédemment développé pour le dosage de type compétitif.

Dans un premier temps, les inventeurs ont vérifié que les anticorps anti-estradiol obtenus reconnaissaient le composé non silylé (réaction croisée de 53% avec les anticorps par rapport à l'estradiol) et non le composé silylé. La vérification était positive.

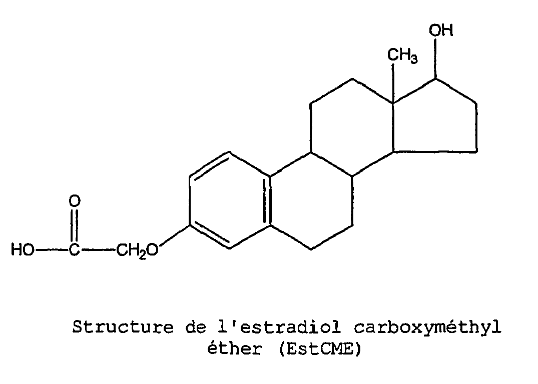

Ils ont ensuite testé l'accrochage covalent de l'estradiol (fixation) sur deux types de plaques: des plaques possédant des groupements aminés (plaques Nunc-NH₂), et des plaques activées avec de la polylysine (poly-Lys).

### 3) Tests de l'accrochage du dérivé d'estradiol

L'EstCME a été incubé sur les plaques NUNC en présence de concentrations équimolaires de N-hydroxysuccinimide (NHS) - un agent permettant la liaison entre les groupements aminés de la plaque et les groupements carboxylés de l'antigène - pendant 3 heures à 22°C. La révélation a été effectuée avec un anticorps anti-estradiol 100 ng/ml pendant 4 heures à 20°C, puis par un anticorps de chèvre anti-anticorps de lapin couplé à l'acétylcholinestérase (CAS-AchE à 2 unités Ellman/ml) pendant 2 heures à 22°C. L'activité enzymatique est révélée par ajout du réactif d'Ellman, un substrat de l'AchE.

Les résultats obtenus sont reportés sur la figure 10 annexée. Ils montrent que la fixation de l'estradiol est effective, et le maximum semble atteint à la plus haute concentration testée (100 µg/ml).

Cette expérience initiale a été reproduite avec des plaques sur lesquelles a été préalablement absorbée de la polylysine (poly-Lys)(entre 1 et 100 µg/ml en tampon phosphate).

Les résultats obtenus sont reportés sur la figure 11 annexée. Dans ce cas, les résultats sont du même ordre quel que soit la concentration de polylysine étudiée. Ceci indique par ailleurs que les groupements aminés portés par la polylysine sont plus accessibles pour la fixation de l'estradiol choisi.

Afin d'étudier plus particulièrement l'influence de la concentration en polylysine, le test a été répété en utilisant une gamme plus large de concentration en polylysine. Les résultats obtenus sont reportés sur le graphe de la figure 12 annexée. Ils montrent un optimum de polylysine autour de 1 µg/ml.

Les deux figures précédentes (figures 11 et 12) semblent indiquer un couplage effectif du dérivé de l'estradiol sur les plaques avec un plateau obtenu pour une concentration de polylysine de 1 µg/ml et une concentration d'estradiol carboxyméthyl éther de 10 µg/ml.

### 4) Silylation du dérivé d'estradiol

Par ailleurs, les inventeurs ont cherché à montrer les meilleures conditions de silylation du composé ci-dessus. L'estradiol carboxyméthyl éther a été mis à réagir avec du MTBSTFA à diverses concentrations (1/5, 1/2, et 1/1) et deux températures (22° ou 37°C) de 0 à 60 minutes. La mesure de la silylation est réalisé au moyen du dosage compétitif de l'estradiol silylé dans lequel l'estradiol silylé perd sa reconnaissance (et dont le signal doit augmenter).

Les résultats obtenus sont reportés sur la figure 13 annexée. Cette figure montre que dans des conditions de forte concentration en MTBSFA, l'estradiol carboxyméthyl éther perd de son immunoréactivité. Cette perte est matérialisée par une augmentation de signal due à l'absence de reconnaissance. Un effet dose du MTBSFA est démontré.

L'ensemble de ces résultats - efficacité du couplage sur la phase solide et de la silylation - ont permis d'effectuer le test de couplage/désilylation sur plaques suivant.

### 5) Test de couplage/désilylation

L'estradiol carboxyméthyl éther (8,3 mg/ml) a été mis à réagir en présence de MTBSTFA à la même concentration pendant 1 heure à 22°C, puis dilué en eau à 1 µg/ml et mis à réagir sur des plaques revêtues de la polylysine (1 µg/ml) pendant 1 heure à 22°C en présence de NHS.

Une désilylation a été effectuée en l'absence ou en présence de HF à la concentration 1 mM pendant 1 heure à 22, 37 ou 64°C.

Les résultats sont reportés sur la figure 14 annexée. Comme le montre cette figure, on peut observer une augmentation de l'immunoréactivité qui correspond à une désilylation de l'estradiol carboxyméthyl éther silylé en présence de HF.

La détection de HF par le procédé de l'invention est donc démontrée lorsque le composé organique silylé est fixé sur un support.

### LISTE DE REFERENCES

**[1]** International Society for Fluoride Research, Fluoride **31(2)**, 1998, 74-80 - disponible sur le site Internet http://www.fluoride-journal.com/98-31-2/31274-80.htm.
**[2]** Occupational Safety and Health Administration", http://www.osha-dlc.gov/dts/sltc/methods/inorganic/id110/id110.html.
**[3]** Alexei Vasiliev et al, Elsevier, Analytica chimica acta, Sensor and Actuators, B **49** (1998), 133-138.
**[4]** http://www.bionics-instrument.com/p_fluorine.htm.
**[5]** Ezan E et al, Pharmcokinetics in healthy volunteers and patients of NAc-SDKP (seraspenide), a negative regulator of hematopoiesis, Drug Metab Dispos 1994 Nov-Dec; **22**(6): 843-848.
**[6]** Shi RZ et al., Development of an enzyme-linked immunosorbent assay with monoclonal antibody for quantification of homovanillic acid [corrected] in human urine samples, Clin Chem 1998 Aug; **44**(8 Pt 1):1674-1679.
**[7]** Taran F et al., Competitive enzyme immunoassay for urinary vanillylmandelic acid, Clin Chim Acta 1997 Aug 29; **264**(2):177-192.
**[8]** Boschelli et al, Tetrahedron Letters **26**, 5329-5242,1985.
**[9]** Machard S et al., A sensitive amphotericin B immunoassay for pharmacokinetic and distribution studies, Antimicrob Agents Chemother 2000 Mar; **44**(3) :546-550.
**[10]** Cleary JD et al., Amphotericin B enzyme-linked immunosorbent assay, Antimicrob Agents Chemother 1996 Mar; **40**(3):637-641.
**[11]** Fitzgerald RL, Herold DA., Serum total testosterone: immunoassay compared with negative chemical ionization gas chromatography-mass spectrometry, Clin Chem 1996 May; **42**(5):749-755.
**[12]** Luceri F et al., Gas chromatography-mass spectrometry measurement of 6-beta-OH-cortisol/cortisol ratio in human urine: a specific marker of enzymatic induction, Clin Chem Lab Med 2001 Dec;**39**(12):1234-1249
**[13]** Munro CJ et al., Relationship of serum estradiol and progesterone concentrations to the excretion profiles of their major urinary metabolites as measured by enzyme immunoassay and radioimmunoassay, Clin Chem 1991 Jun; **37**(6):838-844.
**[14]** Metaye T et al., Comparative measurement of progesterone receptors in breast cancer by biochemical and immunoenzymatic assays, Ann Biol Clin (Paris) 1990; **48**(10) :732-736
**[15]** Foekens JA et al., Comparison of enzyme immunoassay and dextran-coated charcoal techniques for progesterone receptor determination in human breast cancer cytosols, J Steroid Biochem 1988 Jun; **29**(6):571-574.
**[16]** Hosoda H et al., Sensitivity of steroid enzyme immunoassays. Comparison of four label enzymes in an assay system using a monoclonal anti-steroid antibody, Chem Pharm Bull (Tokyo) 1989 Jul; **37**(7):1834-1837
**[17]** Hubl W et al., An improved solid-phase enzyme and luminescent immunoassay system for steroid hormones and digoxin, Clin Chem 1988 Dec; **34**(12):2521-2523
**[18]** Hocart CH et al, Mass spectrometry and chromatography of t-butyldimethylsilyl derivatives of cytokinin bases, Cytokine Anal Biochem 1986 Feb 15; **153**(1):85-96.
**[19]** Garcia de Salamone IE et al., Cytokinin production by plant growth promoting rhizobacteria and selected mutants, Can J Microbiol 2001 May; **47**(5):404-411.
**[20]** Trione EJ et al., A quantitative fluorescence enzyme immunoassay for plant cytokinins, Anal Biochem 1987 Apr; **162**(1):301-308.
**[21]** Steffenrud S et al., Gas chromatography-mass spectrometry of monohydroxyeicosatetraenoic acids as their methyl esters trimethylsilyl, allyldimethylsilyl and tert.-butyldimethylsilyl ethers, J Chromatogr 1987 May 15; **416**(2):219-235.
**[22]** Smith BJ et al, Measurement of plasma prostaglandin E2 using capillary gas chromatography negative ion chemical ionization mass spectrometry, Res Commun Chem Pathol Pharmacol 1983 Apr; **40**(1):73-86.
**[23]** David Percival M, Continuous spectrophotometric assay amenable to 96-well plate format for prostaglandin E synthase activity, Anal Biochem 2003 Feb 15; **313**(2):307-310.
**[24**] Hoffman SW et al., A reliable and sensitive enzyme immunoassay method for measuring 8-isoprostaglandin F2 alpha: a marker for lipid peroxidation after experimental brain injury, Neurosci Methods 1996 Oct; **68**(2):133-136
**[25]** Knapp RD 1979 Handbook of analytical derivatization reactions New York John Willey and sons.
**[26]** Lau HL et al, 1966, J Gas Chromatography 4, 136.
**[27]** Tallent WH, et al., Bis(trimethylsilyl)acetamide in the silylation of lipolysis products for gas-liquid chromatography, J Lipid Res 1968 Jan; **9**(1):146-148
**[28]** Mawhinney TP et al., Gas-liquid chromatography and mass spectral analysis of mono-, di- and tricarboxylates as their tert.-butyldimethylsilyl derivatives, J Chromatogr 1986 Jun 27; 361:117- .
**[29]** Mawhinney TP et al., Simultaneous determination of N-acetylglucosamine, N-acetylgalactosamine, N-acetylglucosaminitol and N-acetylgalactosaminitol by gas-liquid chromatography, J Chromatogr 1986 Jan 3; **351**(1):91-102
**[30]** Bazan AC and Knapp DR, Impived derivative of 6-ketoprostaglandin F1 for gas chromatography-mass spectrometriuc analysis, J Chromatography 1982, **236,** 201-207.
**[31]** Choi MH et al., Determination of estrone and 17 beta-estradiol in human hair by gas chromatography-mass spectrometry, Analyst 2000 Apr; **125**(4):711-714.
**[32]** Dehennin L, Estradiol-17 beta determined in plasma by gas chromatography-mass spectrometry with selected ion monitoring of mixed silyl ether-perfluoroacyl ester derivatives and use of various stable-isotope-labeled internal standards, Clin Chem 1989 Apr; **35**(4):532-.
**[33]** Andersson SH et al., Analysis of profiles of unconjugated steroids in rat testicular tissue by gas chromatography-mass spectrometry, J Steroid Biochem 1985 Oct; **23**(4):469-.
**[34]** Ishikawa E et al., Development and applications of sensitive enzyme immunoassay for antibodies: a review, J Clin Lab Anal 1989; **3**(4):252-265.
**[35]** Ishikawa E, Development and clinical application of sensitive enzyme immunoassay for macromolecular antigens-a review, Clin Biochem 1987 Dec; **20**(6):375-385.
**[36]** Oellerich M, Enzyme-immunoassay: a review, J Clin Chem Clin Biochem 1984 Dec; **22**(12): 895-904.
**[37]** O'Sullivan MJ et al., Enzyme immunoassay: a review, Ann Clin Biochem 1979 Sep; **16**(5):221-240.
**[38]** EZAN E et al, Strategies for developing specific and sensitive radioimmunoassays. In: Handbook of Pharmacology: Radioimmunoassay in basic and clinical pharmacology (Patrono C. and Beskar P., eds) **82**(1987)143-179.
**[39]** Buscarlet L et al., Cross-linking of 17 beta-estradiol to monoclonal antibodies by direct UV irradiation: application to an enzyme immunometric assay, Anal Chem 1999 Mar 1; **71**(5):1002-1008.
**[40]** Nakagomi M et al., Enzyme immunoassay for the measurement of 17-alpha-estradiol 17-N-acetylglucosaminide in rabbit urine, Steroids 1999 Apr; **64**(4):301-307.
**[41]** el Jabri J, Enzyme immunoassay for plasma estradiol using a monoclonal antibody, J Steroid Biochem Mol Biol 1991 Mar; **38**(3):339-343.
**[42]** Dhar TK et al., Homogeneous enzyme immunoassay of estradiol using estradiol-3-O-carboxymethyl ether as hapten, Steroids 1988 May-Jun; **51**(5-6):519-526.
**[43]** ELLMAN G.L. et al., (1961) Biochem. Pharmacol. **7**, 88.
**[44]** EP-A-0139552.
**[45]** US 5,047,300.

## Revendications

1. Procédé de détection et/ou de dosage de fluorure (F⁻) ou de fluorure d'hydrogène (HF) dans un échantillon comprenant les étapes suivantes:
- mettre en contact en solution aqueuse ledit échantillon avec un composé organique silylé pour obtenir une solution de mesure, ledit composé organique silylé étant désilylé lorsqu'il est en présence d'acide fluorhydrique ou de fluorure, le composé organique silylé et le composé organique désilylé pouvant être détectés et/ou dosés distinctement l'un de l'autre; et
- détecter et/ou doser dans ladite solution de mesure l'apparition du composé organique désilylé ou la disparition du composé organique silylé qui a lieu si du fluorure ou du fluorure d'hydrogène est présent dans l'échantillon.

2. Procédé selon la revendication 1, dans lequel le composé organique silylé est de formule : 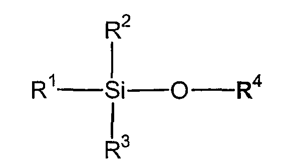 dans laquelle R¹, R² et R³ sont choisis indépendamment parmi les alkyles en C₁ à C₆, et R⁴ est ledit composé organique.

3. Procédé selon la revendication 2, dans lequel R¹, R² et R³ sont choisis indépendamment dans le groupe constitué du méthyle, de l'éthyle, du propyle et du butyle.

4. Procédé selon l'une des revendications 1, 2 ou 3, dans lequel le composé organique est un composé hydroxylé ayant un poids moléculaire de 250 à 200000 g.mol⁻¹.

5. Procédé selon la revendication 1 ou 2, dans lequel le composé organique est un composé hydroxylé choisi dans le groupe constitué de l'estradiol, des peptides, de l'acide homovanillique, de l'amphotéricine, des stéroïdes, des cytokines, de l'acide arachidonique, ou des dérivés de ceux-ci.

6. Procédé selon la revendication 1, dans lequel la détection et/ou le dosage dans ladite solution de mesure de l'apparition du composé organique désilylé ou de la disparition du composé organique silylé est réalisé par chromatographie en phase gazeuse.

7. Procédé selon l'une quelconque des revendications 1 à 6, dans lequel la détection et/ou le dosage dans ladite solution de mesure de l'apparition du composé organique désilylé ou de la disparition du composé organique silylé est réalisé au moyen d'un test immunologique en utilisant un ou plusieurs anticorps dirigé(s) soit contre le composé organique non silylé ou désilylé soit contre le composé organique silylé.

8. Procédé selon la revendication 7, dans lequel le ou les anticorps sont des anticorps monoclonaux.

9. Procédé selon la revendication 7, dans lequel le test immunologique est un immunodosage de type compétitif ou de type non compétitif.

10. Procédé selon la revendication 1 ou 7, dans lequel le composé organique est de l'estradiol ou un de ses dérivés.

11. Procédé selon la revendication 1 ou 7, dans lequel le composé organique est choisi dans le groupe constitué par l'estratriène-1, 3, 5 diol-3, 17µ ou 17µ, et leurs dérivés.

12. Procédé selon l'une quelconque des revendications précédentes, dans lequel le composé organique silylé est utilisé à une concentration de 1 à 2000 ng/ml dans l'étape de mise en contact.

13. Procédé selon l'une quelconque des revendications précédentes, dans lequel le pH de la solution aqueuse de mise en contact est tamponnées à pH 4,5 à 5,5.

14. Procédé selon l'une quelconque des revendications précédentes, dans lequel la mise en contact est réalisée à une température de 54 à 64°C.

15. Trousse de détection et/ou de dosage de fluorure (F⁻) ou de fluorure d'hydrogène (HF) dans un échantillon comprenant les réactifs suivants: un composé organique silylé qui est désilylé lorsqu'il est en présence de fluor ou d'acide fluorhydrique ; et un moyen permettant de détecter l'apparition du composé organique désilylé ou la disparition du composé organique silylé en solution aqueuse.

16. Trousse selon la revendication 15, dans laquelle le composé organique silylé est de formule: 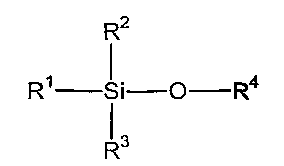 dans laquelle R¹, R² et R³ sont choisis indépendamment parmi les alkyles en C₁ à C₆, et R⁴ est ledit composé organique.

17. Trousse selon la revendication 16, dans laquelle R¹, R² et R³ sont choisis indépendamment dans le groupe constitué du méthyle, de l'éthyle, du propyle et du butyle.

18. Trousse selon l'une des revendications 15, 16 ou 17, dans laquelle le composé organique est un composé hydroxylé ayant un poids moléculaire de 250 à 200000 g.mol⁻¹.

19. Trousse selon la revendication 15 ou 16, dans laquelle le composé organique est un composé hydroxylé choisi dans le groupe constitué de l'estradiol, des peptides, de l'acide homovanillique, de l'amphotéricine, des stéroïdes, des cytokines, de l'acide arachidonique, ou des dérivés de ceux-ci.

20. Trousse selon la revendication 15 ou 16, dans laquelle le composé organique est de l'estradiol ou un de ses dérivés.

21. Trousse selon la revendication 15, dans laquelle le composé organique est choisi dans le groupe constitué par l'estratriène-1, 3, 5 diol-3, 17µ ou 17µ, ou leurs dérivés.

22. Trousse selon l'une quelconque des revendications 15 à 21, comprenant en outre un ou plusieurs anticorps dirigé(s) contre le composé organique non silylé ou désilylé, ou contre le composé organique silylé.

23. Trousse selon la revendication 22, comprenant en outre des réactifs et anticorps nécessaires à la mise en oeuvre d'un immunodosage de type compétitif ou des réactifs et anticorps nécessaires à la mise en oeuvre d'un immunodosage de type non compétitif.

24. Trousse selon la revendication 15, 22 ou 23 comprenant en outre une barrette de polystyrène dans laquelle sont formés un ou plusieurs puits servant de réceptacle(s) pour l'étape de mise en contact et/ou de détection et/ou dosage.

25. Trousse selon la revendication 24, dans laquelle des puits sont recouverts d'anticorps dirigés contre des anticorps de souris anti-estradiol.

26. Trousse selon la revendication 24, dans laquelle de l'estradiol silylé est fixé au fond des puits.

## Claims

1. A method for detecting and/or measuring the concentration of fluoride (F⁻) or hydrogen fluoride (HF) in a sample, comprising the following steps:
- bringing into contact, in aqueous solution, said sample with a silylated organic compound in order to obtain a measurement solution, with said silylated organic compound being desilylated when it is in the presence of hydrofluoric acid or of fluoride, with the silylated organic compound and the desilylated organic compound being able to be detected and/or measured separately from each other; and
- detecting and/or measuring, in said measurement solution, the appearance of the desilylated organic compound, or the disappearance of the silylated organic compound, which takes place if fluoride or hydrogen fluoride is present in the sample.

2. The method as claimed in claim 1, in which the formula of the silylated organic compound is: 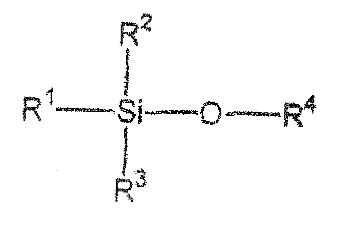 in which R¹, R² and R³ are independently selected from C₁ to C₆ alkyls and R⁴ is said organic compound.

3. The method as claimed in claim 2, in which R¹, R² and R³ are independently selected from the group consisting of methyl, ethyl, propyl and butyl.

4. The method as claimed in one of claims 1, 2 or 3, in which the organic compound is a hydroxylated compound having a molecular weight of from 250 to 200 000 g.mol⁻¹.

5. The method as claimed in claim 1 or 2, in which the organic compound is a hydroxylated compound selected from the group consisting of estradiol, peptides, homovanillic acid, amphotericin, steroids, cytokines, arachidonic acid or derivatives thereof.

6. The method as claimed in claim 1, in which the detection and/or measurement, in said measurement solution, of the appearance of the desilylated organic compound, or of the disappearance of the silylated organic compound, is carried out by means of gas chromatography.

7. The method as claimed in any one of claims 1 to 6, in which the detection and/or the measurement, in said measurement solution, of the appearance of the desilylated organic compound, or of the disappearance of the silylated organic compound, is carried out by means of an immunological test using one or more antibodies which is/are directed either against the unsilylated or desilylated organic compound or against the silylated organic compound.

8. The method as claimed in claim 7, in which the antibody(ies) is/are (a) monoclonal antibody(ies).

9. The method as claimed in claim 7, in which the immunological test is a competitive-type or non-competitive-type immunoassay.

10. The method as claimed in claim 1 or 7, in which the organic compound is estradiol or one of its derivatives.

11. The method as claimed in claim 1 or 7, in which the organic compound is selected from the group consisting of estra-1,3,5-triene-3,17µ or 17y-diol, or" their derivatives.

12. The method as claimed in any one of the preceding claims, in which the silylated organic compound is used at a concentration of from 1 to 2000 ng/ml in the contacting step.

13. The method as claimed in any one of the preceding claims, in which the pH of the contacting aqueous solution is buffered to pH 4.5 to 5.5.

14. The method as claimed in any one of the preceding claims, in which the contacting is effected at a temperature of from 54 to 64°C.

15. A kit for detecting and/or measuring the concentration of fluoride (F⁻) or hydrogen fluoride (HF) in a sample, comprising the following reagents: a silylated organic compound which is desilylated when it is in the presence of fluorine or hydrofluoric acid; and a means for detecting, in aqueous solution, the appearance of the desilylated organic compound or the disappearance of the silylated organic compound.

16. The kit as claimed in claim 15, in which the formula of the silylated organic compound is: 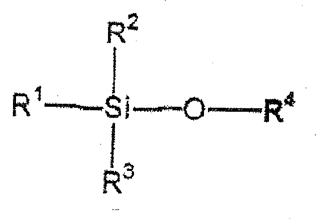 in which R¹, R² and R³ are independently selected from C₁ to C₆ alkyls, and R⁴ is said organic compound.

17. The kit as claimed in claim 16, in which R¹, R² and R³ are independently selected from the group consisting of methyl, ethyl, propyl and butyl.

18. The kit as claimed in one of claims 15, 16 or 17, in which the organic compound is a hydroxylated compound having a molecular weight of from 250 to 200 000 g.mol⁻¹.

19. The kit as claimed in claim 15 or 16, in which the organic compound is a hydroxylated compound selected from the group consisting of estradiol, peptides, homovanillic acid, amphotericin, steroids, cytokines or arachidonic acid, or derivatives thereof.

20. The kit as claimed in claim 15 or 16, in which the organic compound is estradiol or one of its derivatives.

21. The kit as claimed in claim 15, in which the organic compound is selected from the group consisting of estra-1,3,5-triene-3,17µ or 17µ-diol, or their derivatives.

22. The kit as claimed in any one of claims 15 to 21, which additionally comprises one or more antibody(ies) which is/are directed against the unsilylated or desilylated organic compound or against the silylated organic compound.

23. The kit as claimed in claim 22, which additionally comprises reagents and antibodies which are required for implementing a competitive-type immunoassay or reagents and antibodies which are required for implementing a non-competitive-type immunoassay.

24. The kit as claimed in claim 15, 22 or 23, which additionally comprises a polystyrene strip in which is/are formed one or more well(s) which serve(s) as (a) receptacle(s) for the step of contacting and/or detection and/or measurement.

25. The kit as claimed in claim 14, in which wells are coated with antibodies directed against mouse anti-estradiol antibodies.

26. The kit as claimed in claim 24, in which the silylated estradiol is bound to the bottom of the wells.

## Patentansprüche

1. Verfahren zum Nachweis und/oder zur Bestimmung von Fluorid (F⁻) oder Fluorwasserstoff (HF) in einer Probe, wobei das Verfahren die folgenden Stufen umfasst:
- das Inkontaktbringen der Probe in wässriger Lösung mit einer silylierten organischen Verbindung zur Bildung einer Messlösung, wobei die silylierte organische Verbindung in Gegenwart von Fluorwasserstoffsäure oder eines Fluorids desilyliert wird, wobei die silylierte organische Verbindung und die desilylierte organische Verbindung getrennt voneinander nachgewiesen und/oder bestimmt werden können; und
- den Nachweis und/oder die Bestimmung des Auftretens der desilylierten organischen Verbindung oder des Verschwindens der silylierten organischen Verbindung in der Messlösung, das stattfindet, wenn ein Fluorid oder Fluorwasserstoff in der Probe vorhanden ist.

2. Verfahren nach Anspruch 1, bei dem die silylierte organische Verbindung die Formel hat: 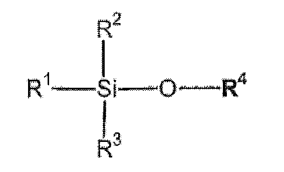 in der R¹, R² und R³ unabhängig voneinander ausgewählt werden aus der Gruppe der C₁-C₆-Alkyle und R⁴ für die genannte organische Verbindung steht.

3. Verfahren nach Anspruch 2, bei dem R¹, R² und R³ unabhängig voneinander ausgewählt werden aus einer Gruppe, die besteht aus Methyl, Ethyl, Propyl und Butyl.

4. Verfahren nach einem der Ansprüche 1, 2 oder 3, bei dem die organische Verbindung eine hydroxylierte Verbindung mit einem Molekulargewicht von 250 bis 200 000 g/mol ist.

5. Verfahren nach Anspruch 1 oder 2, bei dem die organische Verbindung eine hydroxylierte Verbindung ist, die ausgewählt wird aus der Gruppe, die besteht aus Östradiol, Peptiden, Homovanillinsäure, Amphotericin, Steroiden, Cytokinen, Arachidonsäure oder Derivaten davon.

6. Verfahren nach Anspruch 1, in dem der Nachweis und/oder die Bestimmung des Auftretens der desilylierten organischen Verbindung oder des Verschwindens der silylierten organischen Verbindung in der Messlösung durch Gasphasen-Chromatographie durchgeführt wird (werden).

7. Verfahren nach einem der Ansprüche 1 bis 6, bei dem der Nachweis und/oder die Bestimmung des Auftretens der desilylierten organischen Verbindung oder des Verschwindens der silylierten organischen Verbindung in der Messlösung unter Anwendung eines immunologischen Tests durchgeführt wird (werden), bei dem ein oder mehrere Antikörper verwendet werden, die gegen die nicht silylierte oder desilylierte organische Verbindung oder gegen die silylierte organische Verbindung gerichtet sind.

8. Verfahren nach Anspruch 7, bei dem der (die) Antikörper (ein) monoklonale(r) Antikörper ist (sind).

9. Verfahren nach Anspruch 7, bei dem der immunologische Test eine Immunbestimmung vom kompetitiven oder nicht-kompetitiven Typ ist.

10. Verfahren nach Anspruch 1 oder 7, bei dem als organische Verbindung Östradiol oder eines seiner Derivate verwendet wird.

11. Verfahren nach Anspruch 1 oder 7, bei dem die organische Verbindung ausgewählt wird aus der Gruppe, die besteht aus Östra-1,3,5-trien-3,17 µ oder 17 µ-diol und seinen Derivaten.

12. Verfahren nach einem der vorhergehenden Ansprüche, bei dem die silylierte organische Verbindung in einer Konzentration von 1 bis 2000 ng/ml in der Kontaktierstufe verwendet wird.

13. Verfahren nach einem der vorhergehenden Ansprüche, in dem der pH-Wert der wässrigen Lösung in der Kontaktierstufe auf einen Wert von 4,5 bis 5,5 abgepuffert wird.

14. Verfahren nach einem der vorhergehenden Ansprüche, bei dem das Inkontaktbringen bei einer Temperatur von 54 bis 64 °C durchgeführt wird.

15. Kit zum Nachweis und/oder zur Bestimmung von Fluorid (F⁻) oder Fluorwasserstoff (HF) in einer Probe, welcher die folgenden Reagenzien umfasst:
eine silylierte organische Verbindung, die in Gegenwart von Fluor oder Fluorwasserstoffsäure desilyliert wird, und ein Mittel zum Nachweis des Auftretens der desilylierten organischen Verbindung oder des Verschwindens der silylierten organischen Verbindung in wässriger Lösung.

16. Kit nach Anspruch 15, in dem die silylierte organische Verbindung die Formel hat: 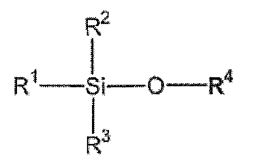 in der R¹, R² und R³ unabhängig voneinander ausgewählt werden aus der Gruppe C₁-C₆-Alkyle und R⁴ die organische Verbindung darstellt.

17. Kit nach Anspruch 16, in dem R¹, R² und R³ unabhängig voneinander ausgewählt werden aus einer Gruppe, die besteht aus Methyl, Ethyl, Propyl und Butyl.

18. Kit nach einem der Ansprüche 15, 16 oder 17, in dem die organische Verbindung eine hydroxylierte Verbindung mit einem Molekulargewicht von 250 bis 200 000 g/mol ist.

19. Kit nach Anspruch 15 oder 16, in dem die organische Verbindung eine hydroxylierte Verbindung darstellt, die ausgewählt ist aus der Gruppe, die besteht aus Östradiol, Peptiden, Homovanillinsäure, Amphotericin, Steroiden, Cytokinen, Arachidonsäure oder Derivaten davon.

20. Kit nach Anspruch 15 oder 16, in dem als organische Verbindung Östradiol oder eines seiner Derivate verwendet wird.

21. Kit nach Anspruch 15, in dem die organische Verbindung ausgewählt ist aus der Gruppe, die besteht aus Östra-1,3,5-trien-3,17 oder 17 µ-diol oder seinen Derivaten.

22. Kit nach einem der Ansprüche 15 bis 21, der außerdem einen oder mehrere Antikörper umfasst, die gegen die nicht-silylierte oder desilylierte organische Verbindung oder gegen die silylierte organische Verbindung gerichtet sind.

23. Kit nach Anspruch 22, der außerdem umfasst Reagenzien und Antikörper, die erforderlich sind, um eine Immunbestimmung vom kompetitiven Typ durchzuführen, oder Reagenzien und Antikörper, die erforderlich sind, um eine Immunbestimmung vom nicht-kompetitiven Typ durchzuführen.

24. Kit nach Anspruch 15, 22 oder 23, der außerdem einen Polystyrol-Stab umfasst, in dem eine oder mehrere Vertiefungen vorgesehen sind, die als AufnahmeBehälter für die Durchführung der Kontaktierstufe und/oder der Nachweis- und/oder Bestimmungsstufe dienen.

25. Kit nach Anspruch 24, in dem die Vertiefungen mit Antikörpern bedeckt sind, die gegen Anti-Östradiol-Maus-Antikörper gerichtet sind.

26. Kit nach Anspruch 24, in dem das silylierte Östradiol am Boden der Vertiefungen fixiert ist.
